# EUROPEAN PATENT SPECIFICATION

(11) **EP 4 463 164 B1**
(45) Date of publication and mention of the grant of the patent: **21.10.2026**
(21) Application number: 23700358.7
(22) Date of filing: 11.01.2023
(51) Int. Cl.: A61K 31/522, A61P 3/00, A61P 9/00, A61P 11/00, A61P 13/12, A61P 17/00, A61P 25/00, A61P 25/28, A61P 27/02, A61P 35/00, A61P 37/00, A61P 43/00, C07D 473/04, C07D 473/06, A61Q 19/08, A61K 8/49

(54) **COMPOUNDS USEFUL IN THE PREVENTION AND/OR TREATMENT OF SENESCENT CELL-RELATED PATHOLOGIES AND DISEASES**
VERBINDUNGEN ZUR VORBEUGUNG UND/ODER BEHANDLUNG VON ERKRANKUNGEN IM ZUSAMMENHANG MIT ALTERNDEN ZELLEN
COMPOSÉS UTILES DANS LA PRÉVENTION ET/OU LE TRAITEMENT DE PATHOLOGIES ET DE MALADIES ASSOCIÉES À DES CELLULES SÉNESCENTES

(30) Priority: 12.01.2022 IT 202200000359
(43) Date of publication of application: 20.11.2024
(73) Proprietor: Istituti Clinici Scientifici Maugeri SpA SB, 27100 Pavia (IT); Consiglio Nazionale Delle Ricerche - CNR, 00185 Roma (IT); INRCA, 60131 Ancona (AN) (IT)
(72) Inventor: GAETANO, Carlo, 27100 Pavia (PV) (IT); ATLANTE, Sandra, 27100 Pavia (PV) (IT); DE ROSA, Maria Cristina, 00100 Roma (RM) (IT); PIROLLI, Davide, 00173 Roma (RM) (IT); FARSETTI, Antonella, 00141 Roma (RM) (IT); MALAVOLTA, Marco, 60128 Ancona (AN) (IT)
(74) Representative: Casci, Tamara
(86) International application number: PCT/IB2023/050262
(87) International publication number: WO 2023/135533

(56) References cited:
- WO-A1-96/36638
- WO-A1-98/22464
- MONTANA J G ET AL: "PDE4 inhibitors: new xanthine analogues", BIOORGANIC & MEDICINAL CHEMISTRY LETTERS, ELSEVIER, AMSTERDAM, NL, vol. 8, no. 20, 20 October 1998 (1998-10-20), pages 2925 - 2930, XP004139587, ISSN: 0960-894X, DOI: 10.1016/S0960-894X(98)00524-1
- DATABASE REGISTRY [online] STN; 29 July 2009 (2009-07-29), - M: "1H-Purine-2,6-dione, 3-(2,4-dimethylphenyl)-3,9-dihydro-1-(phenylmethyl)-", XP055946016, Database accession no. 1170154-96-7
- DATABASE REGISTRY [online] 7 September 2008 (2008-09-07), -: "1H-Purine-2,6-dione, 3-(2,4-dimethylphenyl)-1-(2-furanylmethyl)-3,9- dihydro-", XP055946017, retrieved from STN Database accession no. 1047198-76-4
- DATABASE REGISTRY [online] 24 May 2004 (2004-05-24), - M: "1H-Purine-2,6-dione, 3-(2,6-dimethylphenyl)-3,9-dihydro-1-(phenylmethyl)-", XP055946018, retrieved from STN Database accession no. 685120-33-6
- FUHRMANN-STROISSNIGG HEIKE ET AL: "Identification of HSP90 inhibitors as a novel class of senolytics", vol. 8, no. 1, 1 December 2017 (2017-12-01), XP055926762, Retrieved from the Internet <URL:https://www.nature.com/articles/s41467-017-00314-z.pdf> DOI: 10.1038/s41467-017-00314-z

## Description

### Field of application

The present invention refers to the pharmaceutical field, and it refers in particular to compounds having senolytic activity and their use as a medicament. In particular, the compounds of the invention find application in the prevention and/or treatment of senescent cell-related pathologies and diseases. The present invention also refers to the cosmetic field, and it refers in particular to the cosmetic use of the compounds of the invention in the prevention, reduction and/or treatment of the signs of aging of the skin.

### Prior art

Aging is a risk factor for many chronic diseases. With age, in fact, some cells in the body tissues may enter in a particular stage characterized by a permanent state of replicative arrest and altered transcriptome and proteome. Such cells should not be confused with aged cells as they represent a distinctive cell response (like apoptosis) and are referred to as senescent cells. These cells are no longer able to carry out their normal functions and some of them are also unable to undergo programmed cell death (apoptosis).

Senescent cells thus accumulate in the tissues with age, and secrete harmful substances, such as cytokines, mainly responsible for inflammatory processes, which contribute to age-related diseases, such as for example cardiovascular diseases or disorders, tumours (Muñoz-Espin D, Serrano M., Cellular senescence: from physiology to pathology. Nat Rev Mol Cell Biol. 2014 15:482-96, doi: 10.1038/nrm3823; Childs BG, et al. Cellular senescence in aging and age-related disease: from mechanisms to therapy. Nat Med. 2015 21:1424-35, doi: 10.1038/nm.4000; van Deursen JM. The role of senescent cells in ageing. Nature. 2014 509:439-46, doi: 10.1038/nature13193) and many others.

Cells can also become senescent following exposure to certain environmental conditions, chemical agents, or biological insult, or as a result of disease (Demaria et al. Cellular Senescence Promotes Adverse Effects of Chemotherapy and Cancer Relapse. Cancer Discov. 2017 7:165-176. doi: 10.1158/2159-8290.CD-16-0241; Schafer et al. Cellular senescence mediates fibrotic pulmonary disease. Nat Commun. 2017 8:14532. doi: 10.1038/ncomms14532).

In particular, a senescent cell is associated with enhanced SA-β-gal (senescence-associated β-galactosidase activity) and increase in tumour suppressor proteins such as p16INK4A, p53, and p21. Additionally, there are higher levels of DNA damage, including γ-H2AX and formation of Senescence Associated Heterochromatin Foci (SAHF). Further, senescent cells secrete pro-inflammatory factors, metalloproteinases and other signaling molecules, which are collectively termed as Senescence-Associated Secretory Phenotype (SASP). These SASP molecules have the potential to dissolve intercellular matrix and induce senescence in neighbouring cells (Dutta Gupta S, Pan CH., Recent update on discovery and development of HSP90 inhibitors as senolytic agents. Int J Biol Macromol. 2020 161:1086-1098. doi: 10.1016/j.ijbiomac.2020.06.115).

It has been found that removal of senescent cells during ageing leads to an improvement of several age-related diseases/conditions (Dutta Gupta S, Pan CH. Recent update on discovery and development of HSP90 inhibitors as senolytic agents. Int J Biol Macromol. 2020 161:1086-1098. doi: 10.1016/j.ijbiomac.2020.06.115; Fuhrmann-Stroissnigg et al. Identification of HSP90 inhibitors as a novel class of senolytics. Nat Commun. 2017 8:422. doi: 10.1038/s41467-017-00314-z).

To address age-related diseases, geroprotective drugs are currently available, which interact with the mechanisms of aging, preventing or reversing it and consequently avoiding the formation of senescent cells. Examples of such drugs are rapamycin and metformin. Rapamycin can improve age-related diseases and prolong lifespan and healthspan by inhibiting the mammalian target of rapamycin (mTOR) pathway, which in turn is one of the core and detrimental mechanisms related to aging (Zhang Y. et al. The Role of Rapamycin in Healthspan Extension via the Delay of Organ Aging. Ageing Res Rev. 2021 70:101376. doi: 10.1016/j.arr.2021.101376). Metformin, on the other hand, seems to exert effects on cellular metabolism resulting in anti-hyperglycemic action, enhanced insulin sensitivity, reduction of oxidative stress and additional protective effects on the endothelium and vascular function (Mohammed et al., A Critical Review of the Evidence That Metformin Is a Putative Anti-Aging Drug That Enhances Healthspan and Extends Lifespan. Front Endocrinol Lausanne. 2021 12:718942. doi: 10.3389/fendo.2021.718942).

Senolytic drugs, a subgroup of the geroprotective drugs, have the more specific objective of eliminating the senescent cells.

Senolytic drugs with a diverse range of pharmacological mechanisms are known in the art, such as the combination of dasatinib and quercetin (Zhu Yet et al. The Achilles' heel of senescent cells: from transcriptome to senolytic drugs. Aging Cell. 2015 14:644-58. doi: 10.1111/acel.12344.), navitoclax (Zhu et al. Identification of a novel senolytic agent, navitoclax, targeting the Bcl-2 family of anti-apoptotic factors. Aging Cell. 2016 15:428-35, doi: 10.1111/acel.12445.) and fisetin (Yousefzadeh et al. Fisetin is a senotherapeutic that extends health and lifespan. EBioMedicine. 2018 36:18-28. doi: 10.1016/j.ebiom.2018.09.015).

Targets of senolytic drugs may involve very different pathways, such as the BCL-2-family, the p53 and the PI3K pathway (Zhang et al. Recent advances in the discovery of senolytics. Mech Ageing Dev. 2021 14:111587. doi: 10.1016/j.mad.2021.111587).

The inhibition of the Heat Shock Protein 90 (HSP90) has recently become the object of investigation as a potential strategy for eliminating senescent cells. Potential HSP90 inhibitors that have been identified so far for exhibiting senolytic properties include geldanamycin, ganetespib, alvespimycin and onalespib (Dutta Gupta S, Pan CH. Recent update on discovery and development of HSP90 inhibitors as senolytic agents. Int J Biol Macromol. 2020 161:1086-1098. doi: 10.1016/j.ijbiomac.2020.06.115).

HSP90 is a molecular chaperone that protects polypeptides against various stress conditions such as heat, elevated temperature, exposure to radiation, etc.

To a limited extent, it also assists in folding of nascent polypeptides in normal cells. HSP90 has also been found to be associated to a number of central oncogenic signalling pathways in the cell, and there is ongoing investigation on the inhibition of HSP90 function in the treatment of cancer. There are four homologues of HSP90, i.e., cytosolic HSP90α, HSP90β, organ specific GRP94 (94 kDa glucose-regulated protein found in endoplasmic reticulum) and TRAP1 (tumor necrosis factor (TNF) receptor-associated protein 1 found in mitochondrial matrix).

HSP90 is an ATP-dependent molecular chaperone with dimerisation being important for ATP hydrolysis. The binding of ATP results in the formation of a toroidal dimer structure, in which the two N-terminal domains of HSP90 come into contact with one another and act as a switch in the conformation (Pearl LH, Prodromou C. Structure and in vivo function of Hsp90. Curr Opin Struct Biol. 2000 10:46-51 doi: 10.1016/s0959-440x(99)00047-0). At present, investigations (Sanchez J. et al. Old and new approaches to target the Hsp90 chaperone. Current Cancer Drug Targets. 2020. 20, 4:243-270, doi: 10.2174/1568009619666191202101330) concerning the anticancer properties of HSP90 inhibitory compounds, directed towards the specific target, such as Alvespimycin (17-dimethylaminoethylamino-17-demethoxygeldanamycin (17-DMAG)) or Ganetespib, have not provided any promising results. The failure to obtain regulatory approval for cancer treatment was mainly caused by the cytotoxicity, formulation, and development costs of the candidate compounds. Indeed, most if not all of the randomized controlled trials reported pain as an important adverse effect (Miles VN. et al. The effect of Heat Shock Protein 90 inhibitor on pain in cancer patients: a systematic review and meta-analysis. Medicina, 2021 57:5. doi: 10.3390/medicina57010005). This seems not to be related to HSP90 inhibition but to specific side effects of the compounds. Alvespimycin, for example, displays a relatively low maximum tolerated dose (MTD) of 12 mg/m² (72 mg/Kg) in rats and 8 mg/m² (160 mg/Kg) in dogs (Glaze et al. Preclinical toxicity of a geldanamycin analog, 17-(dimethylaminoethylamino)-17-demethoxygeldanamycin (17-DMAG), in rats and dogs: potential clinical relevance. Cancer Chemother Pharmacol. 2005 56:637-47. doi: 10.1007/s00280-005-1000-9).

The need is therefore felt in the field for a senolytic drug that is effective and that has low toxicity (in particular cytotoxicity).

The technical problem underlying the present invention is thus that of providing compounds that are capable of exerting a senolytic effect, in particular, compounds that are capable of selectively killing senescent cells, over non-senescent cells, in particular senescent cells that are associated with a disease or disorder, more in particular an age-related disease or disorder, or an age-related condition.

Senescent cell-associated diseases or disorders include for example: chronic obstructive pulmonary disease (Baker et al. Senotherapy: A New Horizon for COPD Therapy. Chest. 2020 158:562-570. doi: 10.1016/j.chest.2020.01.027), glaucoma and cataracts (Sreekumar et al. The Emerging Role of Senescence in Ocular Disease. Oxid Med Cell Longev. 2020 2020:2583601. doi: 10.1155/2020/2583601), diabetic pancreas (Aguayo-Mazzucato et al. Acceleration of β Cell Aging Determines Diabetes and Senolysis Improves Disease Outcomes. Cell Metab. 2019 30:129-142.e4. doi: 10.1016/j.cmet.2019.05.006), osteoarthritis (Coryell et al. Nat Rev Rheumatol. 2021 17:47-57. doi: 10.1038/s41584-020-00533-7), atherosclerosis (Childs et al. Senescent intimal foam cells are deleterious at all stages of atherosclerosis. Science. 2016 354:472-477. doi: 10.1126/science.aaf6659), diabetic fat (Sierra-Ramirez et al. Transient metabolic improvement in obese mice treated with navitoclax or dasatinib/quercetin. Aging Albany NY. 2020 12:11337-11348. doi: 10.18632/aging.103607), cancer (Galiana et al. Preclinical antitumor efficacy of senescence-inducing chemotherapy combined with a nanoSenolytic. J Control Release. 2020 323:624-634. doi: 10.1016/j.jconrel.2020.04.045), Alzheimer's disease (Zhang et al. Senolytic therapy alleviates Aβ-associated oligodendrocyte progenitor cell senescence and cognitive deficits in an Alzheimer's disease model. Nat Neurosci. 2019 22:719-728. doi: 10.1038/s41593-019-0372-9.), Parkinson's disease (Chinta et al. Cell Rep. 2018 22:930-940. doi: 10.1016/j.celrep.2017.12.092), and chronic kidney dysfunction (Wang et al. Implication of cellular senescence in the progression of chronic kidney disease and the treatment potencies. Biomed Pharmacother. 2021 135:111191. doi: 10.1016/j.biopha.2020.111191), to name a few.

Age-related conditions include for example the signs of skin ageing.

A further technical problem underlying the present invention is that of providing such compound that exhibits low or no toxicity, in particular cytotoxicity, towards non-senescent cells.

A further technical problem underlying the present invention is that of providing such compound that is simple to produce.

### Summary of the invention

The above technical problem has been solved by the compounds of formula (I) or pharmaceutically acceptable salts or hydrates thereof: 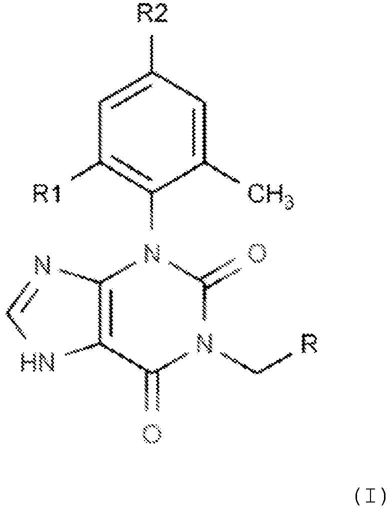
wherein R is chosen from
   - phenyl, a 6-membered heteroaryl group, cyclohexyl, a 5-membered heteroaryl group;
   - a bicyclo [3.1.0] hexanyl group;
   - a C₂-C₅ alkynyl group; and
   - a cubanyl group;
wherein R1 and R2, independently, are chosen from
   - H;
   - C₁-C₆ alkyl (optionally substituted with one or more halogens);
   - C₁-C₆ alkyl-S(O)ₙ-;
   - -CO₂H (or C₁-C₆ alkyl esters thereof or C₁₋₆ alkyl amides thereof);
   - halogen;
   - C₁-C₆ alkoxy;
   - CN;
   - NO₂; and
   - NR₇R₈;
wherein R₇ and R₈, independently, each represent H, C₁-C₆ alkyl, C₁-C₆ alkylcarbonyl, C₁-C₆ alkoxycarbonyl, arylsulphonyl, heteroarylsulphonyl, heterocyclosulphonyl, arylcarbonyl, heteroarylcarbonyl, heterocyclocarbonyl, or C₁-C₆ alkylsulphonyl, or R₇, R₈ and the nitrogen to which they are attached form a 5 or 6 membered heterocyclic ring (such as morpholine or piperidine); and
   - n represents 0-2;
for use in the treatment and/or prevention of a senescence-associated disease or disorder, or for therapeutic use in delaying aging in a subject.

Preferably, in R, the 6-membered heteroaryl group or 5-membered heteroaryl group has 1 or 2 heteroatoms, preferably independently selected from N and S.

Preferably, in R, the 5-membered heteroaryl group is thiazolyl.

Preferably, in R, the 6-membered heteroaryl group is selected from pyridinyl, pyridazinyl and pyrimidinyl.

Preferably, in R, the bicyclo [3.1.0] hexanyl group is a [(1R, 5S)-bicyclo[3.1.0]hexan-3-yl] group.

Preferably, in R, the C₂-C₅ alkynyl group is a propynyl group, more preferably a prop-2-yn-1-yl group.

Preferably, in R, the cubanyl group is a [(2S,3S,4S,5R,7S)-cuban-1-yl] group.

Preferably, R1 and R2 are independently chosen from H and -C₁₋C₆ alkyl (optionally substituted with one or more halogens).

Preferably R1 and R2 are independently chosen from -H, -CH₃ and -CH₂CH₃, more preferably R1 and R2 are both -H. Preferably, R is chosen from:
- phenyl, a 6-membered heteroaryl group, cyclohexyl, a 5-membered heteroaryl group;
- a bicyclo [3.1.0] hexanyl group;
- a C₂-C₅ alkynyl group; and
- a cubanyl group;

more preferably phenyl;
and R1 and R2 are independently chosen from -H, -CH₃ and -CH₂CH₃, more preferably R1 and R2 are both -H.

Preferably, R is chosen from the following groups of formula (a), (b), (c), (d), (e), (f), (g), (h), (i), (l):

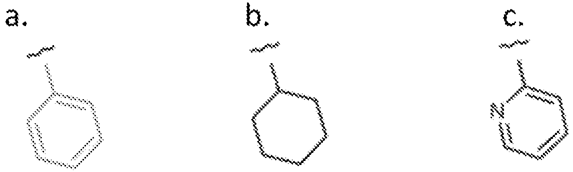

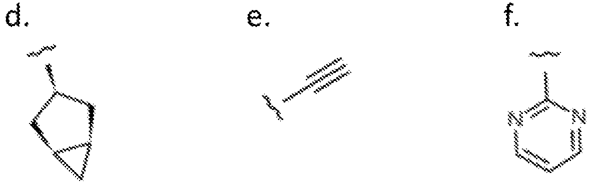

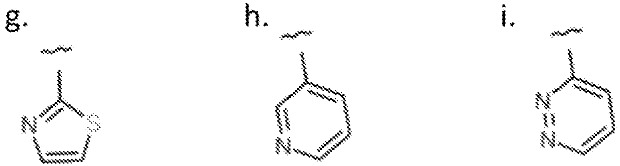

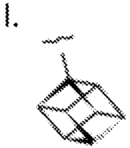

Preferably, R is chosen from the above groups of formula (a), (b), (c), (d), (e), (f), (g), (h), (i), (l), and R1 and R2 are independently chosen from -H, -CH₃ and -CH₂CH₃, more preferably R1 and R2 are both -H.

Based on the above groups for R of formulas (a), (b), (c), (d), (e), (f), (g), (h), (i), (l), the preferred compounds of formula (I) are the following compounds of formula (Ia), (Ib), (Ic), (Id), (Ie), (If), (Ig), (Ih), (Ii), (Il), respectively:

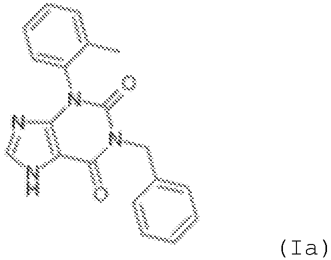

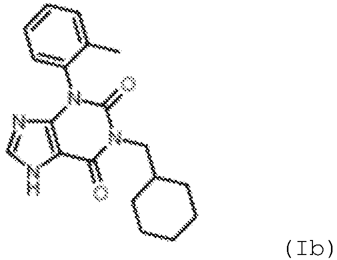

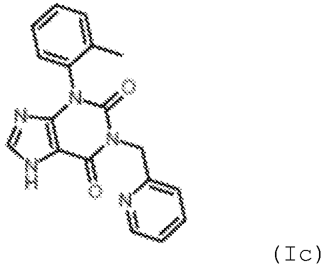

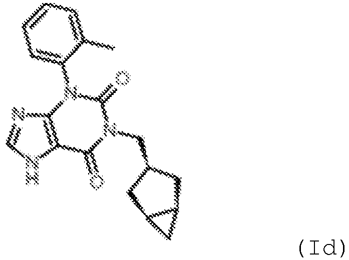

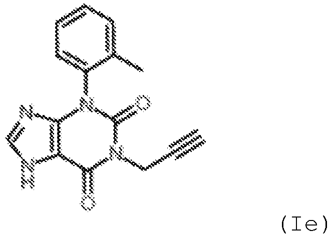

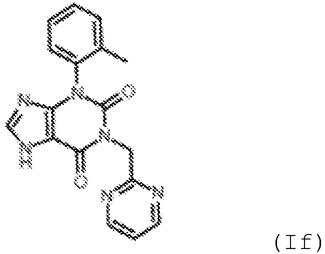

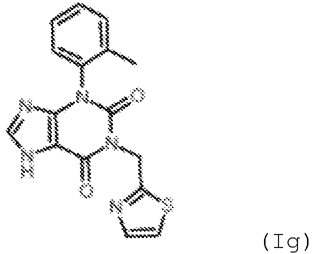

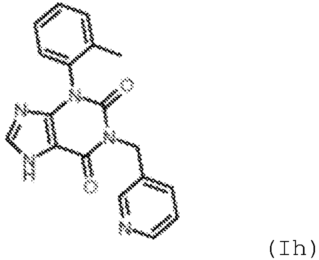

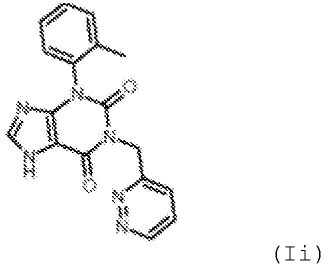

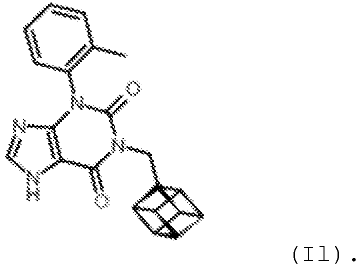

In a preferred embodiment of the invention, R is the group of formula (a):

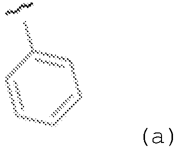

In this preferred embodiment, the preferred compound of formula (I) is the compound of formula (Ia):

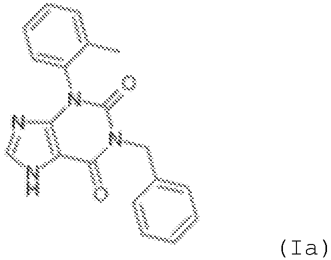

Compound Ia has chemical name 1-benzyl-3-(2-methylphenyl)-2,3,6,7-tetrahydro-1H-purine-2,6-dione, it has MayBridge number KM09711 and zinc code ZINC171079. This compound is identified in the description also with the abbreviation "K5".

Compound Ib has chemical name 1- (cyclohexylmethyl) -3- (2-methylphenyl) -2,3,6, 7-tetrahydro-1H-purine-2,6-dione. Compound Ic has chemical name 3- (2-methylphenyl) -1-[(pyridin-2-yl) methyl]-2,3,6, 7-tetrahydro-1H-purine-2, 6-dione.

Compound Id has chemical name 3- (2-methylphenyl) -1-{ [(1R, 5S)-bicyclo [3.1.0]hexan-3-yl]methyl}-2,3,6, 7-tetrahydro-1H-purine-2, 6-dione.

Compound Ie has chemical name 3- (2-methylphenyl) -1- (prop-2-yn-1-yl) -2, 3,6,7-tetrahydro-1H-purine-2, 6-dione. Compound If has chemical name 3- (2-methylphenyl) -1-[(pyrimidin-2-yl) methyl]-2,3,6, 7-tetrahydro-1H-purine-2, 6-dione.

Compound Ig has chemical name 3- (2-methylphenyl) -1- [(1,3-thiazol-2-yl) methyl]-2, 3,6,7-tetrahydro-1H-purine-2, 6-dione.

Compound Ih has chemical name 3- (2-methylphenyl) -1-[(pyridin-3-yl) methyl]-2,3,6, 7-tetrahydro-1H-purine-2, 6-dione.

Compound Ii has chemical name 3-(2-methylphenyl)-1-[(pyridazin-3-yl)methyl]-2, 3,6,7-tetrahydro-1H-purine-2,6-dione.

Compound Il has chemical name 1-{[(2S, 3S,4S,5R,7S)-cuban-1-yl]methyl}-3-(2-methylphenyl)-2,3,6,7-tetrahydro-1H-purine-2,6-dione.

All the compounds of formula (I) may be in salt form on one of the nitrogen atoms, such as for example chloride, bromide, iodide.

The compounds of formula (I) or pharmaceutically acceptable salts or hydrates thereof of the invention are Heat Shock Protein 90 (HSP90) inhibitors, more preferably HSP90α inhibitors.

The compounds of the invention in fact are compounds which have in common the same pharmacological activity, that is the inhibition of HSP90α protein, in particular of human HSP90α protein [code in data bank UniProtKB-P07900, HS90A_HUMAN].

In the present description under the term "HSP90α inhibitor" a substance is meant capable of inhibiting selectively the activity of the HSP90α protein. Without wishing to be bound by any theory, it is thought that these compounds may be involved in degradation pathways of tumor suppressors or anti-apoptotic molecules in senescent cells.

In another aspect, the present invention refers to a pharmaceutical composition comprising, as active ingredient, at least one compound of formula (I), as above, or pharmaceutically acceptable salts or hydrates thereof, and at least one pharmaceutically acceptable excipient.

Preferably, the pharmaceutical composition is in a form chosen from an aqueous or non-aqueous solution, a suspension, an emulsion (e.g. a microemulsion), a solid, a gel and an aerosol.

In an embodiment of the invention, the pharmaceutical composition is solid, more preferably in the form of a tablet, a capsule, a powder, or any combination thereof. The pharmaceutical composition can be a lyophilized product.

In a preferred embodiment of the invention, the pharmaceutical composition is in the form of an aqueous or non-aqueous solution, a suspension, an emulsion, or any combination thereof, preferably an aqueous or non-aqueous solution or an emulsion.

Preferably, the pharmaceutical composition includes a nanodelivery system, preferably chosen from liposomes, silica nanoparticles, proteolipid nanoparticles.

A nanodelivery system is a delivery system of an active ingredient to its target whereby the active ingredient is associated to a nanoparticle, for example by encapsulation, coating, or chemical bonding.

The preparation of such nanodelivery systems can be carried out according to methods known in the field. Preferably, the pharmaceutical composition is for administration via a route chosen from intraperitoneal, intravenous, intramuscular, transdermal, intradermal, infusion (for example bolus infusion), subcutaneous, oral, enteral, rectal, intranasal, by inhalation, buccal, sublingual, topically, intraocular, vaginal, intracranial injection, and any combination thereof.

In a particularly preferred embodiment, the pharmaceutical composition is for administration via a route chosen from oral, buccal, sublingual and combinations thereof, preferably oral.

Preferably, in the administration via a route chosen from oral, buccal, sublingual and combinations thereof, the composition is in the form of an aqueous solution. It has in fact been found that the oral administration of the pharmaceutical composition of the invention is associated to a greater absorption of the at least one compound of formula (I) or pharmaceutically acceptable salts or hydrates thereof *in vivo.*

In an alternative preferred embodiment, the pharmaceutical composition is for administration via a route chosen from intraperitoneal, intravenous, intramuscular, transdermal, intradermal, infusion, and combinations thereof, preferably intraperitoneal. Preferably, in this latter embodiment, the pharmaceutical composition is in the form of a non-aqueous solution or an emulsion.

Preferably, in this latter embodiment, the pharmaceutically acceptable excipient is a vehicle chosen from oil and lipid emulsion.

Preferably, the oil is pharmaceutical grade seed oil, more preferably corn oil or olive oil.

Preferably, the lipid emulsion is an injectable lipid emulsion, more preferably at a concentration of 10 to 30% (v/v) of the lipid phase in the emulsion.

Preferably the lipid emulsion comprises at least an oil, more preferably chosen from soy oil, corn oil and olive oil, and at least a phospholipid, more preferably an egg yolk phopholipid, even more preferably lecithin.

An example of such injectable lipid emulsion is "Intralipid 20%", produced by Sigma-Aldrich (US).

The pharmaceutical composition of the invention may also comprise one or more excipients chosen from preservatives, stabilisers, dyes, buffers, antioxidants, surfactants and suspending agents.

The pharmaceutical composition may also comprise one or more additional active ingredients.

Preferably, said one or more additional active ingredient is chosen from an active ingredient used in the prevention and/or treatment of any of the senescence-associated diseases or disorders listed below, a geroprotective drug and combinations thereof. Preferably, said geroprotective drug is a senolitic drug, even more preferably chosen from dasatinib, quercetin, navitoclax, fisetin, and combinations thereof.

It can in fact be useful to associate a compound of the present invention with one or more further compounds having geroprotective or senolitic activity. This expedient allows for example the targeting of more than one type of receptor, that is, another receptor, other than HSP90 or HSP90α, and/or allows to prevent and/or treat more than one senescent cell-related disease or disorder.

In a particularly preferred embodiment, the pharmaceutical composition in the form of an aqueous solution for oral administration comprises the at least one compound of formula (I) or pharmaceutically acceptable salts or hydrates thereof, more preferably the compound of formula (Ia) or pharmaceutically acceptable salts or hydrates thereof, at a concentration from 0,5 to 5 mg/ml, more preferably 0.5 to 2 mg/ml, even more preferably about 1 mg/ml.

By way of example, the preparation of such an aqueous solution can be carried out as follows: 0.250 ml of DMSO are added to 5 mg of the compound in a vial and vortexed; after complete dissolution of the compound, 2.375 ml of PEG400 are added and after vortexing 2.375 ml of water are added.

In an alternative preferred embodiment, the pharmaceutical composition is in the form of a non-aqueous solution or an emulsion, for intraperitoneal injection, and comprises the at least one compound of formula (I) or pharmaceutically acceptable salts or hydrates thereof, more preferably the compound of formula (Ia) or pharmaceutically acceptable salts or hydrates thereof, at a concentration from 5 to 25 mg/ml, more preferably from 7 to 15 mg/ml.

The present invention also refers to the pharmaceutical composition comprising, as active ingredient, at least one compound of formula (I), as above, or pharmaceutically acceptable salts or hydrates thereof, and at least one pharmaceutically acceptable excipient, for use as a medicament.

The routes of administration described above for the composition apply to the compounds of the invention.

It has been observed that the admnistration of compound (Ia) enables the crossing of the blood-brain barrier (BBB).

In a preferred embodiment, the compound of formula (I) or pharmaceutically acceptable salts or hydrates thereof, or the pharmaceutical composition, is for administration to a subject in need thereof in a therapy having the duration of six days or less, more preferably from one to four days, even more preferably from one to three days, most preferably about two days. Preferably, the days of administration are consecutive.

In an alternative preferred embodiment, the compound of formula (I) or pharmaceutically acceptable salts or hydrates thereof, or the pharmaceutical composition, is for administration to a subject in need thereof in a therapy having the duration of at least one week, more preferably from one to six weeks, even more preferably from two to four weeks, most preferably about three weeks.

Preferably, in the therapy, in this latter embodiment, the pharmaceutical composition is for administration to a subject in need thereof for one to five, more preferably two to four, even more preferably three to four days per week.

The days of administration per week in this latter embodiment can be consecutive or intermittent. Preferably, the pharmaceutical composition is administered three to four times a week, every other day. That is to say, each day of administration should be followed and preceded by a day without administration. Preferably, the therapy as described above according to either preferred embodiment is repeated, more preferably after a period of one to twelve months, even more preferably two to nine months, even more preferably three to six months, most preferably three to four months, from the end of the previous therapy.

In a preferred embodiment, the therapy as described above is repeated after about three months.

The therapy can be further repeated for an indefinite number of times, as described above.

In a preferred embodiment, the subject in need thereof is a human being, preferably a human being of an age of at least 20 years, more preferably at least 40 years, even more preferably at least 50 years, even more preferably at least 60 years.

The subject in need thereof can also be a non-human animal, more preferably an animal of a class chosen from mammals, fish, amphibians, reptiles and birds, more preferably mammals.

Preferably, the mammals are chosen among those of the genus Felis, Canis, Oryctolagus, Bos, Ovis, Sus, Equus and Mus.

Preferably, the mammals are chosen from domestic mammals and experimental mammals. The domestic mammals are preferably chosen from the species *Felis catus* (cat), *Canis lupus familiaris* (dog), *Oryctolagus cuniculus* (rabbit), *Bos taurus* (cattle, such as cows and bulls), *Ovis aries* (sheep), *Sus domesticus* (swine, such as pig), *Equus ferus* (horse) and *Equus africanus* (donkey). The experimental mammals are preferably *Mus musculus.*

In another aspect, the present invention refers to the compounds of formula (I) or pharmaceutically acceptable salts or hydrates thereof for the use as defined in the claims, as HSP90 inhibitor, more preferably HSP90α inhibitor.

In another aspect, the present invention refers to the pharmaceutical composition comprising at least one compound of formula (I) or pharmaceutically acceptable salts or hydrates thereof and at least one pharmaceutically acceptable excipient for the use as defined in the claims, as HSP90 inhibitor, more preferably HSP90α inhibitor.

In another aspect, the present invention refers to the compounds of formula (I) or pharmaceutically acceptable salts or hydrates thereof for the use as defined in the claims, having the effect of selectively killing senescent cells.

In another aspect, the present invention refers to the pharmaceutical composition comprising at least one compound of formula (I) or pharmaceutically acceptable salts or hydrates thereof and at least one pharmaceutically acceptable excipient for the use as defined in the claims, having the effect of selectively killing senescent cells.

The compounds of the present invention are in fact senolytic agents.

"Senescence" or "senescent cells" as used herein refer to a state wherein cells have acquired one or more markers for senescence in response to some cellular stress. Such marker can include for example permanent withdrawal from the cell cycle, altered methylation, expression of markers for DNA damage, protein and lipid modifications, expression of senescence-associated markers such as beta-galactosidase (SA-β-gal), p16INK41, p53, p21, PAI-1, CD36, Dihomo-15d-PGJ2, DPP4, NKG2D ligands or any one or more SASP factors (e.g. IL-6, MMP3).

A senolytic agent (in the case of the present invention, a compound) is an agent that selectively (preferentially, or to a greater degree) destroys, kills, removes or facilitates selective destruction of senescent cells. In other words, the senolytic agent kills or destroys a senescent cell in a significant manner (biologically, clinically and statistically) compared to its ability to do the same on a non-senescent cell.

Characterizing a compound as a senolytic agent can be accomplished using one or more cell-based assays described herein or in the art. Those of skill in the art will readily appreciate that characterizing a compound as a senolytic agent can be accomplished by comparing the activity of a test agent with appropriate negative and/or positive controls. A senolytic agent reduces (i.e. decreases) percent survival of a plurality of senescent cells (i.e., in some manner reduces the quantity of viable senescent cells in the cell -based assay) compared with one or more negative controls. The source of senescent cells for use in assays may be a primary cell culture, or culture-adapted cell line, including but not limited to, genetically engineered cell lines that may contain chromosomally integrated or episomal recombinant nucleic acid sequences, immortalized or immortalizable cell lines, somatic cell hybrid cell lines, differentiated or differentiable cell lines, transformed cell lines, and the like.

Senescent cells can be detected by techniques and procedures known in the art, for example by way of histochemical or immunohistochemical techniques that detect the senescent marker SA-beta galactosidase (Itahana et al. (2007) Methods to Detect Biomarkers of Cellular Senescence. In: Tollefsbol T.O. (eds) Biological Aging. Methods in Molecular Biology™, vol 371. Humana Press. https://doi.org/10.1007/978-1-59745-361-5_3). Other ways of detecting senescent cells include the detection of other cell-associated molecules, such as p16INK41, p21, PAI-1, CD36, Dihomo-15d-PGJ2, DPP4, NKG2D ligands or any one or more cytokines, chemokines, and proteases known among the SASP factors (e.g. IL-6, MMP3), etc.

Moreover, senescent cells can be detected by morphology (as viewed by microscopy, for example).

Preferably, detection is carried out by detection of the senescent marker SA-beta galactosidase.

The effectiveness of the compounds and compositions of the present invention can be determined by a person skilled in the medical and clinical arts. One or any combination of diagnostic methods, including physical examination, assessment and monitoring of clinical symptoms, and performance of analytical tests and methods may be used for monitoring the health status of the subject.

In another aspect, the present invention refers to the compounds of formula (I) or pharmaceutically acceptable salts or hydrates thereof for use in delaying ageing in a subject.

In another aspect, the present invention refers to the pharmaceutical composition comprising at least one compound of formula (I) or pharmaceutically acceptable salts or hydrates thereof and at least one pharmaceutically acceptable excipient for use in delaying ageing in a subject.

"Delaying ageing in a subject" means herein to slow down or halt the occurrence of one or more of the following phenomena: decline of the immune system, muscle atrophy, decrease in muscle strength, decrease in skin elasticity, delay in wound healing, retinal atrophy, reduced lens transparency, reduced hearing, reduced vision, osteoporosis, sarcopenia, hair graying, cognitive impairment.

In another aspect, the present invention refers to the compounds of formula (I) or pharmaceutically acceptable salts or hydrates thereof for use in the treatment and/or prevention of a senescence-associated disease or disorder.

In another aspect, the present invention refers to the pharmaceutical composition comprising at least one compound of formula (I) or pharmaceutically acceptable salts or hydrates thereof and at least one pharmaceutically acceptable excipient for use in the treatment and/or prevention of a senescence-associated disease or disorder.

A senescence-associated disease or disorder is a disease or disorder associated with an excessive accumulation of senescent cells in the body tissues (such as for example those of the liver, kidney, heart, or pancreas) or a disease or disorder whose progression is associated with an excessive accumulation of senescent cells in the body tissues. It can also be called a senescent cell-associated disease or disorder.

Preferably, the senescence-associated disease or disorder is chosen from a tumour, a metabolic disease, an inflammatory disease or disorder, including an autoimmune disease or disorder, a neurological or neurodegenerative disease or disorder, a pulmonary or respiratory disease or disorder, a proliferative disorder, a renal disorder or disease, a liver disorder or disease, an eye disease or disorder, a cardiovascular disease or disorder, a fibrotic disease or disorder, a viral infection, a dermatological disorder or disease, a geriatric disease or disorder, and side effects associated with chemotherapy or radiotherapy. Preferably, the senescence-associated disease or disorder is chosen from the above list, with the exclusion of asthma.

Preferably, the senescence-associated disease or disorder of the present invention is associated to senescent cells that are sensitive to the activity of HSP90, more preferably HSP90α.

It follows that the compounds of the invention, being HSP90 inhibitors, are effective towards the senescent cells of said senescence-associated diseases or disorders.

Preferably the tumour is a malignant tumour and is chosen from a solid tumour and a liquid tumour, more preferably a solid tumour.

The word "cancer" is also used herein to refer to a malignant tumour.

The solid tumour is preferably chosen from lung cancer, breast cancer, prostate cancer, colorectal cancer, renal cancer, hepatocellular carcinoma, and metastatic melanoma.

The liquid tumour (also referred to as blood cancer) is preferably chosen from hematologic malignancies occurring in one or more chosen from blood, bone marrow and lymph nodes. More preferably, the liquid tumour is chosen from leukaemia, lymphomas and myelomas. Preferably, the metabolic disease is chosen from diabetes, diabetic ulcer, diabetic pancreas, diabetic fat, metabolic syndrome and obesity.

Preferably, the diabetes is Type II diabetes.

Preferably, the inflammatory (including autoimmune) disease or disorder is chosen from osteoarthritis, osteoporosis, chronic arthritis, oral mucositis, inflammatory bowel disease, kyphosis, herniated intervertebral disc, autoimmune encephalomyelitis, rheumatoid arthritis, systemic lupus erythematosus and autoimmune bullous skin diseases (such as for example epidermolysis bullosa acquisita (EBA), bullous pemphigoid (HP), and dermatitis herpetiformos (DH)). Preferably, osteoporosis is chosen from post-menopausal osteoporosis and senile osteoporosis.

Preferably, the neurological or neurodegenerative disease or disorder is chosen from Alzheimer's disease, Parkinson's disease, Huntington's disease, dementia, mild cognitive impairment, dementia with Lewy bodies, ataxia telangiectasia, amyotrophic lateral sclerosis (ALS), macular degeneration, and motor neuron dysfunction.

Preferably, the pulmonary or respiratory disease or disorder is chosen from pulmonary fibrosis, chronic obstructive pulmonary disease, cystic fibrosis, emphysema, bronchiectasis, and age-related loss of pulmonary function.

Preferably, the proliferative disorder is a lymphoproliferative disorder.

Preferably, the renal disorder or disease is chosen from chronic kidney disease, kidney failure and glomerulonephritis.

Preferably, the liver disorder or disease is chronic liver cirrhosis.

Preferably, the eye disease or disorder is chosen from macular degeneration, glaucoma, cataracts, presbyopia, and vision loss.

Preferably, the cardiovascular disease or disorder is chosen from atherosclerosis, coronary artery disease, heart failure, cardiomyopathy, hypertension, myocardial infarction, endocarditis, aortic aneurysm, angina, arrhythmia, congestive heart failure, peripheral vascular diseases, ischemic cardiopathy, and valvular diseases without ischemia.

Preferably, the fibrotic disease or disorder is chosen from idiopathic pulmonary fibrosis, cardiac fibrosis, liver fibrosis, pancreatic fibrosis, and renal fibrosis. Preferably, the viral infection is chosen from Human immunodeficiency virus, hepatitis C virus, Chikungunya virus, lymphocytic choriomeningitis virus, Sendai virus, Kaposi's sarcoma-associated herpesvirus, senescence inducing viral infections, and SARS.

Preferably, the viral infection is SARS, more preferably Covid-19 syndrome.

Preferably, the dermatological disorder or disease is chosen from age related dermatological disorder or disease, chronic wounds, eczema, psoriasis, hyperpigmentation, nevi, rashes, autoimmune bullous skin diseases (such as for example epidermolysis bullosa acquisita (EBA), bullous pemphigoid (HP), and dermatitis herpetiformos (DH)).

Preferably, the geriatric disease or disorder is frailty syndrome or sarcopenia.

In a particularly preferred embodiment of the invention, the senescence-associated disease or disorder is chosen from metabolic syndrome, chronic kidney disease, Type II diabetes, frailty syndrome, sarcopenia, Alzheimer's disease, Parkinson's disease, chronic obstructive pulmonary disease, idiopathic pulmonary fibrosis, cataract, glaucoma, post-menopausal osteoporosis, senile osteoporosis, chronic arthritis, heart failure, coronary artery disease, atherosclerosis, colorectal cancer, breast cancer, prostate cancer, lung cancer, hepatocellular carcinoma, side effects of chemotherapy, senescence inducing viral infections, chronic liver cirrhosis, chronic wounds, age-related dermatological disease or disorder, ALS (amyothrophic lateral sclerosis), more preferably chosen from metabolic syndrome, chronic kidney disease, Type II diabetes, frailty syndrome, sarcopenia, chronic obstructive pulmonary disease, idiopathic pulmonary fibrosis, cataract, glaucoma, post-menopausal osteoporosis, senile osteoporosis, heart failure, coronary artery disease, atherosclerosis, colorectal cancer, breast cancer, prostate cancer, lung cancer, hepatocellular carcinoma, side effects of chemotherapy, senescence inducing viral infections, chronic liver cirrhosis, chronic wounds, age-related dermatological disease or disorder, ALS.

The above listed diseases and disorders are in fact known to be associated to an accumulation of senescent cells in the affected tissues. The following Table 1 shows the effect of such accumulation in each listed disease, and a bibliographic reference.

**Table 1. Effect of accumulation in known senescence-associated diseases or disorders**

| **Pathology/syndrom/condition** | **Role of accumulation of senescent cells** | **Ref no.** |
|---|---|---|
| Metabolic syndrome | worsening or | 1 |
| | progression | |
| Chronic kidney disease | initiation and progression | 2 |
| Type II diabetes | initiation and progression of insulin resistance | 3 |
| Frailty syndrome/sarcopenia | worsening and progression | 4 |
| Alzheimer's disease | initiation and progression | 5 |
| Parkinson's disease | initiation and progression | 6 |
| Chronic obstructive pulmonary disease | initiation and progression | 7 |
| Idiopathic pulmonary fibrosis | initiation and progression | 8 |
| Cataract | initiation and progression | 9 |
| Glaucoma | progression | 10 |
| Post-menopausal or senile osteoporosis | initiation and progression | 11 |
| Chronic arthritis | progression | 12 |
| Heart failure | initiation and progression | 13 |
| Coronary artery disease | initiation and progression | 14 |
| Atherosclerosis | Initiation, progression, plaque instability | 15 |
| Colorectal cancer | promotion, worsening or progression, bad response to therapy | 16 |
| Breast cancer | promotion, worsening or progression | 17 |
| Prostate cancer | worsening or progression | 18 |
| Lung cancer | prognostic, cancer relapse, metastasis | 19 |
| Hepatocellular carcinoma | promotion, progression | 20 |
| Side effects of chemotherapy | recurrence of chemotreated cancers, ulcers, hyposalivation, chemotherapy-induced fatigue | 21 |
| Senescence inducing viral infections | persistence of infection | 22 |
| Chronic liver cirrhosis | progression | 23 |
| Chronic wounds | promotion, progression | 24 |
| Age related dermatological disorder or disease | promotion, progression | 25 |
| ALS (amyothrophic lateral sclerosis) | promotion, progression | 26 |

The numbers in the column "Ref no." of Table 1 refer to the following articles:
1) Palmer AK, Xu M, Zhu Y, Pirtskhalava T, Weivoda MM, Hachfeld CM, Prata LG, van Dijk TH, Verkade E, Casaclang-Verzosa G, Johnson KO, Cubro H, Doornebal EJ, Ogrodnik M, Jurk D, Jensen MD, Chini EN, Miller JD, Matveyenko A, Stout MB, Schafer MJ, White TA, Hickson LJ, Demaria M, Garovic V, Grande J, Arriaga EA, Kuipers F, von Zglinicki T, LeBrasseur NK, Campisi J, Tchkonia T, Kirkland JL. Targeting senescent cells alleviates obesity-induced metabolic dysfunction. Aging Cell. 2019 Jun;18(3):e12950. doi: 10.1111/acel.12950.
2) Xu J, Zhou L, Liu Y. Cellular Senescence in Kidney Fibrosis: Pathologic Significance and Therapeutic Strategies. Front Pharmacol. 2020 Dec 11;11:601325. doi: 10.3389/fphar.2020.601325.
3) Narasimhan A, Flores RR, Robbins PD, Niedernhofer LJ. Role of Cellular Senescence in Type II Diabetes. Endocrinology. 2021 Oct 1;162(10):bqab136. doi: 10.1210/endocr/bqab136.
4) Zampino M, Ferrucci L, Semba RD. Biomarkers in the path from cellular senescence to frailty. Exp Gerontol. 2020 Jan;129:110750. doi: 10.1016/j.exger.2019.110750.
5) Zhang P, Kishimoto Y, Grammatikakis I, Gottimukkala K, Cutler RG, Zhang S, Abdelmohsen K, Bohr VA, Misra Sen J, Gorospe M, Mattson MP. Senolytic therapy alleviates Aβ-associated oligodendrocyte progenitor cell senescence and cognitive deficits in an Alzheimer's disease model. Nat Neurosci. 2019 May;22(5):719-728. doi: 10.1038/s41593-019-0372-9.
6) Verma DK, Seo BA, Ghosh A, Ma SX, Hernandez-Quijada K, Andersen JK, Ko HS, Kim YH. Alpha-Synuclein Preformed Fibrils Induce Cellular Senescence in Parkinson's Disease Models. Cells. 2021 Jul 5;10(7):1694. doi: 10.3390/cells10071694.
7) Araya J, Kuwano K. Cellular senescence-an aging hallmark in chronic obstructive pulmonary disease pathogenesis. Respir Investig. 2022 Jan;60(1):33-44. doi: 10.1016/j.resinv.2021.09.003.
8) Kellogg DL, Kellogg DL Jr, Musi N, Nambiar AM. Cellular Senescence in Idiopathic Pulmonary Fibrosis. Curr Mol Biol Rep. 2021;7(3):31-40. doi: 10.1007/s40610-021-00145-4.
9) Gulluni F, Prever L, Li H, Krafcikova P, Corrado I, Lo WT, Margaria JP, Chen A, De Santis MC, Cnudde SJ, Fogerty J, Yuan A, Massarotti A, Sarijalo NT, Vadas O, Williams RL, Thelen M, Powell DR, Schueler M, Wiesener MS, Balla T, Baris HN, Tiosano D, McDermott BM Jr, Perkins BD, Ghigo A, Martini M, Haucke V, Boura E, Merlo GR, Buchner DA, Hirsch E. PI(3,4)P2-mediated cytokinetic abscission prevents early senescence and cataract formation. Science. 2021 Dec 10;374(6573):eabk0410. doi: 10.1126/science.abk0410.
10) Rocha LR, Nguyen Huu VA, Palomino La Torre C, Xu Q, Jabari M, Krawczyk M, Weinreb RN, Skowronska-Krawczyk D. Early removal of senescent cells protects retinal ganglion cells loss in experimental ocular hypertension. Aging Cell. 2020 Feb;19(2):e13089. doi: 10.1111/acel.13089.
11) Farr JN, Rowsey JL, Eckhardt BA, Thicke BS, Fraser DG, Tchkonia T, Kirkland JL, Monroe DG, Khosla S. Independent Roles of Estrogen Deficiency and Cellular Senescence in the Pathogenesis of Osteoporosis: Evidence in Young Adult Mice and Older Humans. J Bone Miner Res. 2019 Aug;34(8):1407-1418. doi: 10.1002/jbmr.3729.
12) Xu M, Bradley EW, Weivoda MM, Hwang SM, Pirtskhalava T, Decklever T, Curran GL, Ogrodnik M, Jurk D, Johnson KO, Lowe V, Tchkonia T, Westendorf JJ, Kirkland JL. Transplanted Senescent Cells Induce an Osteoarthritis-Like Condition in Mice. J Gerontol A Biol Sci Med Sci. 2017 Jun 1;72(6):780-785. doi: 10.1093/gerona/glw154.
13) Yan C, Xu Z, Huang W. Cellular Senescence Affects Cardiac Regeneration and Repair in Ischemic Heart Disease. Aging Dis. 2021 Apr 1;12(2):552-569. doi: 10.14336/AD.2020.0811.
14) Latorre E, Pilling LC, Lee BP, Bandinelli S, Melzer D, Ferrucci L, Harries LW. The VEGFA156b isoform is dysregulated in senescent endothelial cells and may be associated with prevalent and incident coronary heart disease. Clin Sci (Lond). 2018 Feb 2;132(3):313-325. doi: 10.1042/CS20171556.
15) Childs BG, Baker DJ, Wijshake T, Conover CA, Campisi J, van Deursen JM. Senescent intimal foam cells are deleterious at all stages of atherosclerosis. Science. 2016 Oct 28;354(6311):472-477. doi: 10.1126/science.aaf6659.
16) Guo Y, Ayers JL, Carter KT, Wang T, Maden SK, Edmond D, Newcomb P P, Li C, Ulrich C, Yu M, Grady WM. Senescence-associated tissue microenvironment promotes colon cancer formation through the secretory factor GDF15. Aging Cell. 2019 Dec;18(6):e13013. doi: 10.1111/acel.13013.
17) Di GH, Liu Y, Lu Y, Liu J, Wu C, Duan HF. IL-6 secreted from senescent mesenchymal stem cells promotes proliferation and migration of breast cancer cells. PLoS One. 2014 Nov 24;9(11):e113572. doi: 10.1371/journal.pone.0113572.
18) Fiard G, Stavrinides V, Chambers ES, Heavey S, Freeman A, Ball R, Akbar AN, Emberton M. Cellular senescence as a possible link between prostate diseases of the ageing male. Nat Rev Urol. 2021 Oct;18(10):597-610. doi: 10.1038/s41585-021-00496-8.
19) Domen A, Deben C, De Pauw I, Hermans C, Lambrechts H, Verswyvel J, Siozopoulou V, Pauwels P, Demaria M, van de Wiel M, Janssens A, Hendriks JMH, Van Schil P, Vermorken JB, Vandamme T, Prenen H, Peeters M, Lardon F, Wouters A. Prognostic implications of cellular senescence in resected non-small cell lung cancer. Transl Lung Cancer Res. 2022 Aug;11(8):1526-1539. doi: 10.21037/tlcr-22-192.
20) Kang TW, Yevsa T, Woller N, Hoenicke L, Wuestefeld T, Dauch D, Hohmeyer A, Gereke M, Rudalska R, Potapova A, Iken M, Vucur M, Weiss S, Heikenwalder M, Khan S, Gil J, Bruder D, Manns M, Schirmacher P, Tacke F, Ott M, Luedde T, Longerich T, Kubicka S, Zender L. Senescence surveillance of pre-malignant hepatocytes limits liver cancer development. Nature. 2011 Nov 9;479(7374):547-51. doi: 10.1038/nature10599.
21) Demaria M, O'Leary MN, Chang J, Shao L, Liu S, Alimirah F, Koenig K, Le C, Mitin N, Deal AM, Alston S, Academia EC, Kilmarx S, Valdovinos A, Wang B, de Bruin A, Kennedy BK, Melov S, Zhou D, Sharpless NE, Muss H, Campisi J. Cellular Senescence Promotes Adverse Effects of Chemotherapy and Cancer Relapse. Cancer Discov. 2017 Feb;7(2):165-176. doi: 10.1158/2159-8290.CD-16-0241.
22) Malavolta M, Giacconi R, Brunetti D, Provinciali M, Maggi F. Exploring the Relevance of Senotherapeutics for the Current SARS-CoV-2 Emergency and Similar Future Global Health Threats. Cells. 2020 Apr 8;9(4):909. doi: 10.3390/cells9040909.
23) Wiemann SU, Satyanarayana A, Tsahuridu M, Tillmann HL, Zender L, Klempnauer J, Flemming P, Franco S, Blasco MA, Manns MP, Rudolph KL. Hepatocyte telomere shortening and senescence are general markers of human liver cirrhosis. FASEB J. 2002 Jul;16(9):935-42. doi: 10.1096/fj.01-0977com.
24) Wilkinson HN, Hardman MJ. Senescence in Wound Repair: Emerging Strategies to Target Chronic Healing Wounds. Front Cell Dev Biol. 2020 Aug 11;8:773. doi: 10.3389/fcell.2020.00773.
25) Fitsiou E, Pulido T, Campisi J, Alimirah F, Demaria M. Cellular Senescence and the Senescence-Associated Secretory Phenotype as Drivers of Skin Photoaging. J Invest Dermatol. 2021 Apr;141(4S):1119-1126. doi: 10.1016/j.jid.2020.09.031.
26) Das, M.M., Svendsen, C.N. Astrocytes show reduced support of motor neurons with aging that is accelerated in a rodent model of ALS. Neurobiol. Aging 2015, 36, 1130-1139.

Preferably, the treatment and/or prevention of a senescence-associated disease or disorder has the effect of selectively killing senescent cells.

Preferably, the treatment and/or prevention of a senescence-associated disease or disorder has the effect of delaying ageing in a subject.

Preferably, the treatment and/or prevention of a senescence-associated disease or disorder comprises the administration of a therapeutically effective amount of at least one compound of formula (I) or pharmaceutically acceptable salts or hydrates thereof, as above, or of the pharmaceutical composition comprising at least one compound of formula (I) or pharmaceutically acceptable salts or hydrates thereof and at least one pharmaceutically acceptable excipient, as above, to a subject in need thereof.

"Therapeutically effective amount", as used herein, refers to the amount of a compound that, when administered to a subject for treating a disease or disorder, is sufficient to effect such treatment for the disease or disorder.

The "therapeutically effective amount" will vary depending on the compound, the disease or disorder and its severity and the age, weight, etc., of the subject to be treated.

In a preferred embodiment, the treatment and/or prevention of a senescence-associated disease or disorder comprises said administration to a subject in need thereof in a therapy having the duration of six days or less, more preferably from one to four days, even more preferably from one to three days, most preferably about two days. Preferably, the days of administration are consecutive.

In an alternative preferred embodiment, the treatment and/or prevention of a senescence-associated disease or disorder comprises said administration to a subject in need thereof in a therapy having the duration of at least one week, more preferably from one to six weeks, even more preferably from two to four weeks, most preferably about three weeks.

Preferably, in this latter embodiment, said administration is carried out for one to five, more preferably two to four, even more preferably three to four days per week.

The days of administration per week in this latter embodiment can be consecutive or intermittent. Preferably, the admnistration is carried out three to four times a week, every other day. That is to say, each day of administration should be followed and preceded by a day without administration.

Preferably, the therapy as described above according to either preferred embodiment is repeated, more preferably after a period of one to twelve months, even more preferably two to nine months, even more preferably three to six months, most preferably three to four months, from the end of the previous therapy.

In a preferred embodiment, the therapy as described above is repeated after about three months.

The therapy can be further repeated for an indefinite number of times, as described above.

In a preferred embodiment, the subject in need thereof is a human being, preferably a human being of an age of at least 20 years, more preferably at least 40 years, even more preferably at least 50 years, even more preferably at least 60 years.

The subject in need thereof may also be a non-human animal, more preferably an animal of a class chosen from mammals, fish, amphibians, reptiles and birds, more preferably mammals.

Preferably, the mammals are chosen among those of the genus Felis, Canis, Oryctolagus, Bos, Ovis, Sus, Equus and Mus.

Preferably, the mammals are chosen from domestic mammals and experimental mammals. The domestic mammals are preferably chosen from the species *Felis catus* (cat), *Canis lupus familiaris* (dog), *Oryctolagus cuniculus* (rabbit), *Bos taurus* (cattle, such as cows and bulls), *Ovis aries* (sheep), *Sus domesticus* (swine, such as pig), *Equus ferus* (horse) and *Equus africanus* (donkey).The experimental mammals are preferably Mus musculusPreferably, the treatment and/or prevention of a senescence-associated disease or disorder comprises the administration of an effective amount of at least one compound of formula (I) or pharmaceutically acceptable salts or hydrates or thereof, as above, or of the pharmaceutical composition comprising at least one compound of formula (I) or pharmaceutically acceptable salts or hydrates thereof and at least one pharmaceutically acceptable excipient, as above, to a subject in need thereof, in association with one or more additional active ingredient, as described above.

The at least one compound of formula (I) or pharmaceutically acceptable salts or hydrates thereof, as above, or pharmaceutical composition comprising at least one compound of formula (I) or pharmaceutically acceptable salts or hydrates thereof and at least one pharmaceutically acceptable excipient, as above, can be administered consecutively, simultaneously, or sequentially with the one or more other active ingredients.

The compounds of formula (I) or pharmaceutically acceptable salts or hydrates thereof of the invention can be prepared according to methods known in the field and described in the literature. Use may also be made of variants which are known in the field.

All the compounds of formula (I) can be prepared, for example, through the synthetic methodology shown in Synthesis Scheme 1, below.

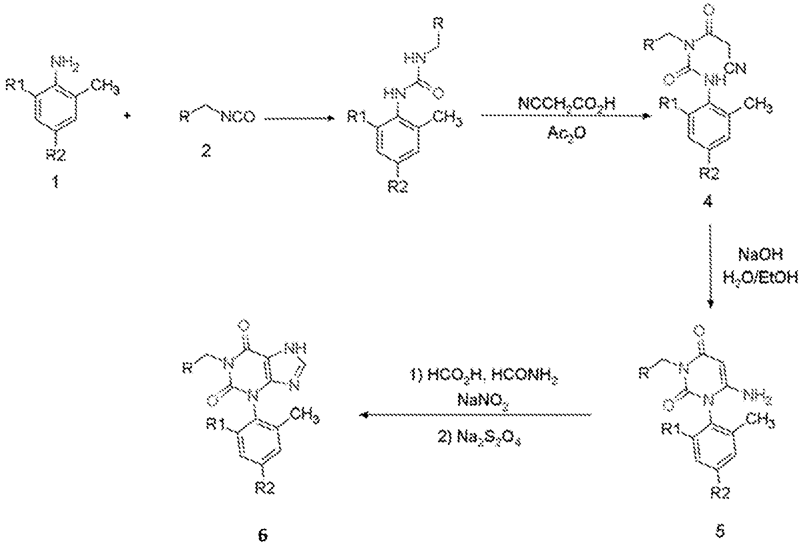

### Synthesis Scheme 1

In synthesis scheme 1, Ac₂O is acetic anhydride; EtOH is ethanol.

Compounds 1 and 2 can be purchased, for example by CAS numbers 95-53-4 and 3173-56-6, respectively (see paragraphs below with reference to Synthesis Scheme 2), for example from Sigma-Aldrich (US) (www.sigmaaldrich.com).

The compound of formula (Ia), herein also abbreviated to "K5", can be prepared as shown in Synthesis Scheme 2, below.

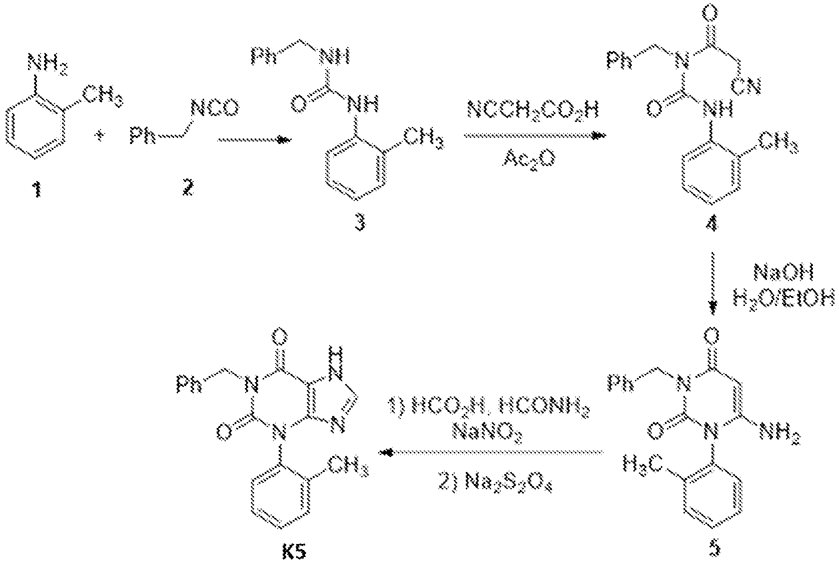

### Synthesis Scheme 2

In Synthesis Scheme 2, Ac₂O is acetic anhydride; EtOH is ethanol.

Compounds 1 and 2 can be purchased by CAS numbers 95-53-4 and 3173-56-6, respectively, for example from Sigma-Aldrich (US) (www.sigmaaldrich.com).

More in particular, condensation of o-toluidine (1) (CAS number 95-53-4) with benzyl isocyanate (2) (CAS number 3173-56-6) gives urea (3) whose acylation with cyanoacetic acid, provides acylurea (4). Hydrolysis of the cyano group, followed by in situ cyclization, yields aminouracyl (5) (see Papesch V, Schroder E.F., J. Org. Chem. 1951, 16, 1879; Ohtsuka Y., Bull. Soc. Chim. Jap. 1973, 46, 506). Nitrosation of (5) and reduction (sodium dithionite/formamide) of the corresponding nitroso derivative to amine and reaction of the resulting diamine intermediate with formic acid, gives the compound of formula (Ia) (K5) (see Brodereck H., Edenhofer A., Chem. Berichte 1955, 1306; Montana J.G. et al., Bioorg. Med. Chem. 1998, 8, 2925).

The compound of formula (Ia) can be purchased from MolPort (MolPort, SIA, Riga, Latvia), for example online from the website www.molport.com using identification code MolPort-002-908-949.

In another aspect, the present invention refers to a compound of formula (I) or pharmaceutically acceptable salts or hydrates thereof, as above, excluding the compound of formula (Ia) and optionally also pharmaceutically acceptable salts or hydrates thereof.

Preferably, in R, the 6-membered heteroaryl group or 5-membered heteroaryl group has 1 or 2 heteroatoms, preferably independently selected from N and S.

Preferably, in R, the 5-membered heteroaryl group is thiazolyl.

Preferably, in R, the 6-membered heteroaryl group is selected from pyridinyl, pyridazinyl and pyrimidinyl.

Preferably, in R, the bicyclo [3.1.0] hexanyl group is a [(1R, 5S)-bicyclo[3.1.0]hexan-3-yl] group.

Preferably, in R, the C₂-C₅ alkynyl group is a propynyl group, more preferably a prop-2-yn-1-yl group.

Preferably, in R, the cubanyl group is a [(2S, 3S, 4S, 5R, 7S) -cuban-1-yl] group.

Preferably, R1 and R2 are independently chosen from H and -C₁₋C₆ alkyl (optionally substituted with one or more halogens).

Preferably, R1 and R2 are independently chosen from -H, -CH₃ and -CH₂CH₃, more preferably R1 and R2 are both -H.

Preferably, R is chosen from:
- phenyl, a 6-membered heteroaryl group, cyclohexyl, a 5-membered heteroaryl group;
- a bicyclo [3.1.0] hexanyl group;
- a C₂-C₅ alkynyl group; and
- a cubanyl group;

more preferably phenyl;
and R1 and R2 are independently chosen from -H, -CH₃ and -CH₂CH₃, more preferably R1 and R2 are both -H.

Preferably, R is chosen from the groups of formula (b), (c), (d), (e), (f), (g), (h), (i), (1), as above. Preferably, R is chosen from the above groups of formula (b), (c), (d), (e), (f), (g), (h), (i), (1), and R1 and R2 are independently chosen from -H, -CH₃ and -CH₂CH₃, more preferably R1 and R2 are both -H.

Based on the above groups for R of formulas (b), (c), (d), (e), (f), (g), (h), (i), (l), the preferred compounds of formula (I) are the above described compounds of formula (Ib), (Ic), (Id), (Ie), (If), (Ig), (Ih), (Ii), (Il), respectively, or pharmaceutically acceptable salts or hydrates thereof.

Prodrug derivatives of the compounds of formula (I) or pharmaceutically acceptable salts or hydrates thereof as above, excluding compound (Ia) and optionally also pharmaceutically acceptable salts or hydrates thereof, also fall within the scope of the present invention.

In another aspect, the present invention refers to a kit comprising one or more of the compounds of formula (I) or pharmaceutically acceptable salts or hydrates thereof, as above, optionally with the exclusion of compound (Ia) and optionally also pharmaceutically acceptable salts or hydrates thereof, or the pharmaceutical composition, comprising, as active ingredient, at least one compound of formula (I), as above, or pharmaceutically acceptable salts or hydrates thereof, and at least one pharmaceutically acceptable excipient as above, and one or more additional active ingredient, as above.

In another aspect, the present invention refers to a kit, as above, for the use as defined in the claims, as HSP90 inhibitor, more preferably HSP90α inhibitor; for the use as defined in the claims, in selectively killing senescent cells; for use in delaying ageing in a subject; or for use in the treatment and/or prevention of a senescence-associated disease or disorder.

In another aspect, the present invention refers to a method for preparing a compound of formula (I) or pharmaceutically acceptable salts or hydrates thereof, as above, the method comprising the steps of Synthesis Scheme 3:

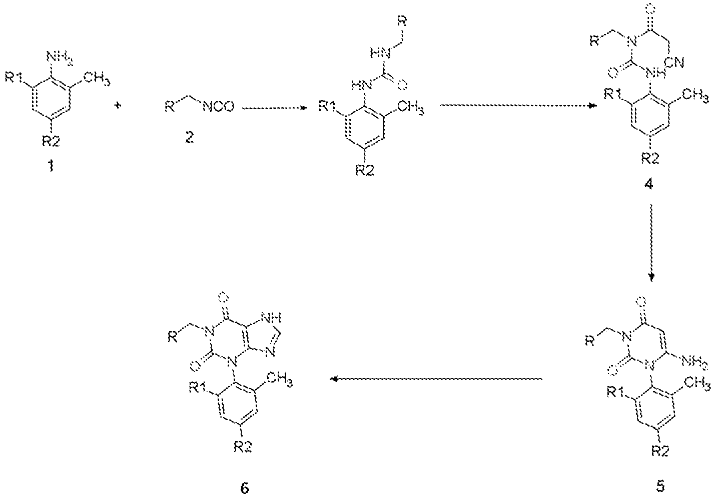

### Synthesis Scheme 3

Preferably, the method comprises the steps of synthesis shown in Synthesis Scheme 1, above, even more preferably the steps of synthesis shown in Synthesis Scheme 4, below, even more preferably the steps of synthesis shown in Synthesis Scheme 2, above.

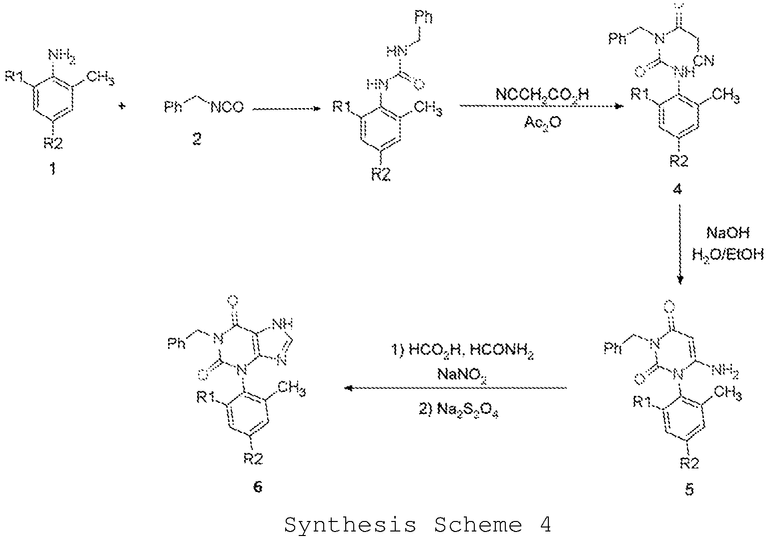

In another aspect, the present description describes a method for selectively killing senescent cells that comprises administering a therapeutically-effective amount of at least one compound of formula (I) or pharmaceutically acceptable salts or hydrates thereof, as above, or of the pharmaceutical composition comprising at least one compound of formula (I) or pharmaceutically acceptable salts or hydrates thereof and at least one pharmaceutically acceptable excipient, as above,to a subject in need thereof.

In another aspect, the present description describes a method for delaying ageing in a subject that comprises administering a therapeutically-effective amount of at least one compound of formula (I) or pharmaceutically acceptable salts or hydrates thereof, as above, or of the pharmaceutical composition comprising at least one compound of formula (I) or pharmaceutically acceptable salts or hydrates thereof and at least one pharmaceutically acceptable excipient, as above, to a subject in need thereof.

In another aspect, the present description describes a method for treating and/or preventing a senescence-associated disease or disorder that comprises the step of administering a therapeutically-effective amount of one or more compounds of formula (I) or pharmaceutically acceptable salts or hydrates thereof, as above, or of the pharmaceutical composition comprising at least one compound of formula (I) or pharmaceutically acceptable salts or hydrates thereof and at least one pharmaceutically acceptable excipient, as above to a subject in need thereof.

As mentioned above, current investigations concerning the anticancer properties of HSP90 inhibitory compounds, directed towards a specific target, such as Alvespimycin (17-DMAG) or Ganetespib, have not provided satisfactory results. The high cytotoxicity of the compounds is one of the main reasons for their failure to obtain regulatory approval.

The Applicant has now surprisingly found that the compounds of formula (I) or pharmaceutically acceptable salts or hydrates thereof according to the invention are capable of exerting a senolytic effect, i.e. of selectively killing senescent cells, and of doing so with low or no cytotoxicity phenomena. Such compounds thus represent an optimal solution in the prevention and/or treatment of senescent cell-associated diseases such as those described above.

As can be seen from the Examples below, in fact, a selected number of compounds was tested for senolytic activity and toxicity.

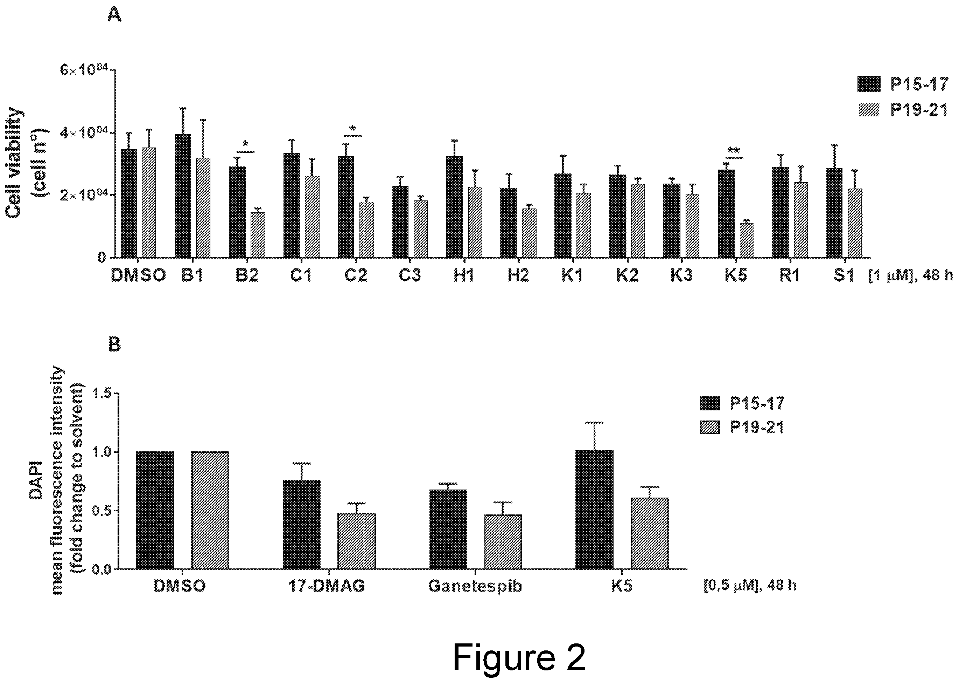

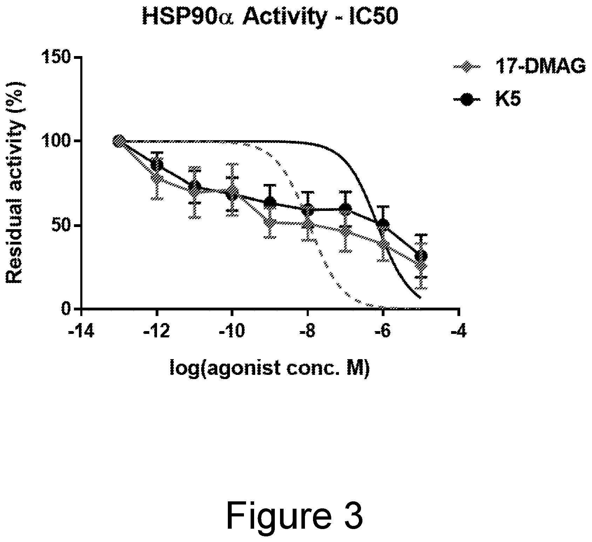

The *in vitro* test that was used for this purpose is based on the comparison of the observed effect of different candidate compounds on a model based on primary human fetal lung cells IMR90 in serial passages (up to P21), designed to mimic cell ageing. The growth curves shown in Figure 2A show that a compound according to the invention (Ia, or "K5") acts selectively and significantly, in the cells, at passages between P19 and P21, without showing particular cytotoxic effects when compared to cells at early passages (P15-P17), as confirmed in the tests summarised in Figure 2B and Figure 3.

Compounds (Ib) to (Il) are derivatives of K5, which have shown to have comparable binding affinity to HSP90α compared to K5.

As it is envisaged that the compounds of the invention could be administered in a therapy that is short-term, and possibly intermittent, any toxic impact of the compounds would be further lowered.

Moreover, as the compounds of the present invention exhibit an inflammation-reducing effect, it is also envisaged that the administration of the compounds of the present invention to a subject under polytherapy (i.e. a subject who is taking more than one drug on a regular basis) for example for the treatment of chronic inflammatory conditions, would have the effect of enabling a reduction of the dosages of one or more of the other drugs taken by the subject.

In another aspect, the present invention refers to the cosmetic use of a compound of formula (I), as above, or cosmetically acceptable salts or hydrates thereof, to prevent, reduce and/or treat the signs of ageing of the skin.

Herein, the term "cosmetic" is to be interpreted as "non-therapeutic".

Preferably, in R, the 6-membered heteroaryl group or 5-membered heteroaryl group has 1 or 2 heteroatoms, preferably independently selected from N and S. Preferably, in R, the 5-membered heteroaryl group is thiazolyl.

Preferably, in R, the 6-membered heteroaryl group is selected from pyridinyl, pyridazinyl and pyrimidinyl. Preferably, in R, the bicyclo [3.1.0] hexanyl group is a [(1R, 5S)-bicyclo[3.1.0]hexan-3-yl] group.

Preferably, in R, the C₂-C₅ alkynyl group is a propynyl group, more preferably a prop-2-yn-1-yl group. Preferably, in R, the cubanyl group is a [(2S,3S,4S,5R,7S)-cuban-1-yl] group.

Preferably, R1 and R2 are independently chosen from H and -C₁₋C₆ alkyl (optionally substituted with one or more halogens).

Preferably, R1 and R2 are independently chosen from -H, -CH₃ and -CH₂CH₃, more preferably R1 and R2 are both -H.

Preferably, R is chosen from:
- phenyl, a 6-membered heteroaryl group, cyclohexyl, a 5-membered heteroaryl group;
- a bicyclo [3.1.0] hexanyl group;
- a C₂-C₅ alkynyl group; and
- a cubanyl group;

more preferably phenyl;
and R1 and R2 are independently chosen from -H, -CH₃ and -CH₂CH₃, more preferably R1 and R2 are both -H.

Preferably, R is chosen from the groups of formula (a), (b), (c), (d), (e), (f), (g), (h), (i), (l), as above.

Preferably, R is chosen from the above groups of formula (a), (b), (c), (d), (e), (f), (g), (h), (i), (1), and R1 and R2 are independently chosen from -H, - CH₃ and -CH₂CH₃, more preferably R1 and R2 are both -H.

Based on the above groups for R of formulas (a), (b), (c), (d), (e), (f), (g), (h), (i), (l), the preferred compounds of formula (I) are the above described compounds of formula (Ia), (Ib), (Ic), (Id), (Ie), (If), (Ig), (Ih), (Ii), (Il), respectively, more preferably compound (Ia).

In another aspect, the present description describes a cosmetic composition comprising at least one compound of formula (I), as above, or cosmetically acceptable salts or hydrates thereof, and at least one cosmetically acceptable excipient.

In another aspect, the present invention refers to the cosmetic use of the cosmetic composition comprising at least one compound of formula (I), as above, or cosmetically acceptable salts or hydrates thereof, and at least one cosmetically acceptable excipient to prevent, reduce and/or treat the signs of ageing of the skin.

Preferably, the signs of ageing of the skin are chosen from loss of elasticity, wrinkling, loss of shine, thinning of the skin, age spots, sagging, stretch marks, broken blood vessels, dryness, and combinations thereof, more preferably loss of elasticity, wrinkling and age spots, most preferably wrinkling.

The skin can be the skin of the face or the skin of the body or parts of the body.

Preferably, the compounds of formula (I), as above, or cosmetically acceptable salts or hydrates thereof, or the cosmetic composition, as above, are for topical administration.

Examples of cosmetic excipients which can be used are polymers of carboxyvinyl type, polyethyleneglycols, propyleneglycol, waxes, fatty substances, esters and triglycerides of fatty acids, stearic derivatives such as glycerol stearate, alcohols such as, for example, stearyl alcohols, cetostearyl alcohols, cetyl alcohol, polyol, polyoxyethylene cetyl ether, vegetable oils such as sweet almond oil, mineral oils such as vaseline oil, glycerine, lanolin derivatives, wetting agents, thickeners, stablizers, emulsifiers, preservatives, perfumes, colorants or other excipients known and currently used, and combinations thereof.

In some embodiments the compositions of the invention can comprise excipients commonly used in the formulation of cosmetic preparations such as preservatives, bactericidal agents, stabilizers, antioxidants, emulsifiers, buffers, humectants, coloring agents and other excipients commonly used in cosmetic preparation techniques, in amounts in line with those provided for common cosmetic formulations.

The cosmetic composition can be in a solid, semisolid or liquid form.

Suitable formulations in solid form include creams, gels, liniments, pastes, ointments, preferably creams. Suitable formulations in liquid form include solutions, suspensions, lotions, gels (such as a hydrogels or lipogels) serums, oil-in-water emulsions, water-in-oil emulsions, preferably a gel.

In the case of formulations in a fluid or semi-fluid form, the compounds of formula (I) can be diluted in a liquid form carrier, for example water.

Preferably, the compounds of formula (I), as above, or cosmetically acceptable salts or hydrates thereof, or the cosmetic composition, are to be applied once or twice a day on an area of skin requiring prevention, reduction and/or treatment of the signs of ageing.

In another aspect, the present invention refers to a cosmetic method for the prevention, reduction and/or treatment of the signs of ageing of the skin comprising the step of applying a cosmetically active amount of at least one compound of formula (I), as above, or cosmetically acceptable salts or hydrates thereof, or the cosmetic composition, as above, on an area of skin requiring prevention, reduction and/or treatment of the signs of ageing.

Without wishing to be bound by theory, it is thought that the compounds of the invention exert their anti-ageing action through their immunomodulatory, antiinflammatory and anti-oxidative action.

### Brief description of the drawings

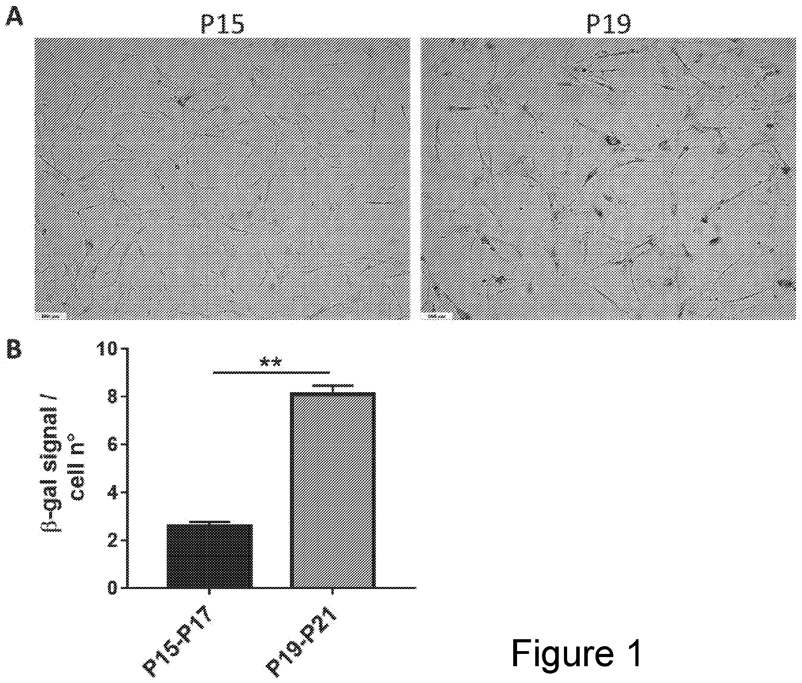

Figure 1 shows: (A) The images showing early-passage cells (P15, left) and advanced-passage cells (P19, right), on which the β-galactosidase assay was performed, according to the supplier protocol (Senescence β-Galactosidase Staining Kit, #9860S, Cell Signaling Technology, Inc., MA, USA). (B) The graph representing the mean quantification of the colorimetric signal of β-galactosidase product, comparing experiments on P15-P17 cells and P19-P21 cells (representative of senescent cells). Microscopy images were obtained using a Leica DMi1 microscope (Leica Microsystems, IL, USA), at magnification 5X and analyzed by ImageJ software, normalized to the number of cells, n = 6. The error bars represent the standard error, ** p <0.005. The data were analyzed using the Mann-Whitney test.
Figure 2 shows: (A) The graph representing the average of the number of cells surviving treatment with 13 compounds, including K5, on the two groups of cell populations P15-P17 and P19-P21, at 1 µM concentration, counted manually by burker chamber, n = 3; and (B) The graph representing the average of the fold changes of the signal emitted by the DAPI, quantified by spectrophotometer, *vs.* solvent. K5 was compared with the two known inhibitors at a concentration of 0,5 µM, n = 3. The error bars represent the standard error, the data were analyzed using two-way ANOVA, followed by Sidak post-hoc test. DMSO (dimethyl sulfoxide) is the control.
Figure 3 is a graph representing the HSP90α activity inhibition curves of K5, compared with the compound 17-DMAG, n=3. Data are reported as residual activity in response to increasing dose of the inhibitor. The data has been analyzed using GraphPad Prism 7 Software, which allowed to extrapolate the sigmoidal curve.

### Detailed description of the invention

The invention is further described by way of the following non-limiting examples.

### Materials and methods

**Cell culture and replicative senescence.** Fetal pulmonary fibroblasts, IMR90, were provided by the European Collection of Authenticated Cell Culture (ECACC) and grown in a complete medium consisting of Minimum Essential Medium (MEM), 10% Fetal Bovine Serum (FBS), 1% penicillin-streptomycin (antibiotic), 1% L-glutamine. Cells with passages from P15 to P21 were used; cells were considered incrementally senescent from passage P19 to passage P21. As can be seen in Figure 1, at P19, the cells were bigger and the beta-galactosidase activity is higher. Figure 1B shows that the ratio of the intensity of the beta-gal ("signal") to the number of cells, increases with the number of passages of the cells.

**β-galactosidase assay** (Itahana et al., (2007) Methods to Detect Biomarkers of Cellular Senescence. In: Tollefsbol T.O. (eds) Biological Aging. Methods in Molecular Biology™, vol 371. Humana Press. https://doi.org/10.1007/978-1-59745-361-5_3). The cells were fixed in paraformaldehyde 2% and incubated with the substrate, X-gal, in a solution at pH 6, at 37°C, overnight (16 h).

The images were acquired using an optical microscope (Leica) with 5X magnification. The images were analyzed using the ImageJ software, quantifying the colorimetric signal emitted by each cell. The mean signal intensity was then normalized for the total number of cells. **Survival curves of the compounds.** To perform survival curves, cells with passages from P15 to P21 were tested at increasing concentrations of 0,5 µM, 1 µM, 10 µM, 25 µM over 24 and 48 h to evaluate the compounds' efficacy and cytotoxicity compared to the solvent (DMSO). The cells were then counted manually, in triplicate, using disposable burker chambers.

For the DAPI staining (4',6-diamidin-2-phenylindol) assay, each compound was tested at increasing concentrations 0,1 µM, 0,5 µM, 1 µM, 10 µM, for 48 h, the reference compounds 17-DMAG and Ganetespib were also used as control of the experiment (Fuhrmann-Stroissnigg et al. Identification of HSP90 inhibitors as a novel class of senolytics. Nat Commun. 2017 8:422 doi: 10.1038/s41467-017-00314-z). Following treatment, the cells were seeded in 96mw. The adherent cells were then fixed with 70% ethanol, incubated for 30 min with a 3 µM DAPI solution; the fluorimetric signal was measured using a multiplate reader (Victor Nivo-Perkin Elmer) set to 350/460 nm (Ligasová A, Koberna K. Quantification of fixed adherent cells using a strong enhancer of the fluorescence of DNA dyes. Sci Rep. 2019 Jun 18; 9(1):8701. doi: 10.1038/s41598-019-45217-9. PMID: 31213648; PMCID: PMC6581942.).

**HSP90 inhibition activity assay.** HSP90-directed inhibitory activity was assessed employing fluorescence polarization measuring competitive binding of fluorescein isothiocyanate-labeled geldanamycin (FITC-geldanamycin) to recombinant HSP90α as a function of test compound concentration using the commercially available HSP90 N-Terminal Domain Assay Kit (BPS Bioscience, Catalog #50293, CA, USA) according to the manufacturer's instructions. Briefly, the compounds were diluted at different concentrations between 1 pM to 10 µM and incubated for 2 h with the enzyme and substrate at RT while stirring. The fluorescence intensity was measured with a multiplate reader (Victor Nivo-Perkin Elmer) set at 485/530 nm. Data have been represented as the percentage of residual enzyme activity relative to the solvent (DMSO). Data were analyzed by GraphPad Prism 7 Software which allowed to extrapolate the sigmoidal curve and calculate the IC50.

**Statistical analysis.** The results were analyzed using GraphPad Prism 7 statistical software to generate graphs showing the mean values of at least three independent experiments ± standard error (SE); for each analysis, a specific comment has been added. The following tests determined significance between experimental groups: non-parametric Mann Whitney and two-way ANOVA, followed by Sidak post-hoc tests to compare the various conditions; The p-value indicates statistical significance: *p < 0.05, **p <; 0.005.

### EXAMPLE 1

### Characterisation of the biological properties of 1-benzyl-3-(2-methylphenyl)-2,3,6,7-tetrahydro-1H-purine-2,6-dione(K5).

13 compounds including K5, with different structures, were assayed using an *in vitro* senescence model, that was developed utilizing serial passages of primary human fetal lung cells IMR90. In this context, the first signs of senescence were found between the nineteenth and twenty-first passage. Senescence was detected using the "Senescence β-Galactosidase Staining Kit" (#9860S, Cell Signaling Technology, Inc., MA, USA). Briefly, cells at different passages were fixed and incubated with the β-Galactosidase substrate at 37°C, overnight in a dry incubator. Then the 13 compounds were tested for their potential senolytic activity on cells between passages 19 and 21, considering cells around passage 15 as control reference (Figure 2A).

The 13 compounds were purchased from MolPort (MolPort, SIA, Riga, Latvia). In particular, K5 was purchased online from the website www.molport.com using identification code MolPort-002-908-949.

K5 has the following structure:

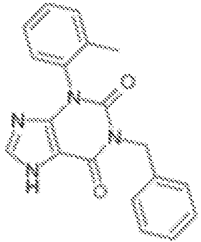

Of the 13 compounds examined, K5 (1-benzyl-3-(2-methylphenyl)-2,3,6,7-tetrahydro-1H-purine-2,6-dione) revealed a marked senolytic capacity, reducing the senescent population by about 50% in the first 48 hours of exposure to the compound (see Figure 2A).

More in detail, growth curves were initially performed, testing each compound in a concentration range from 0.5 to 25 µM for 48 h. Figure 2A shows the differences in cells treated at different passages at 1 µM concentration, respectively. In particular, K5 appeared to act selectively and significantly, in cells at passages between P19 and P21, without showing particular cytotoxic effects when compared to cells at early passages (P15-P17). The reduced cytotoxic effect of K5 is shown by the fact that the younger cells (P15-P17) are not killed by the compounds. The compounds instead selectively kill only senescent cells (P19-P21).

To confirm data obtained by traditional cell counting by a single operator, a quantification method based on UV-visible spectrophotometry was also implemented, exploiting the ability of the DAPI to mark cell nuclei. The cells that survived the action of senolytics were fixed and incubated with the fluorescent indicator. The specific signal was measured using the microplate multimode reader, Victor NIVO (PerkinElmer Tech. GmbH, Germany). Figure 2B shows the effect of the two selected compounds, at 0.5 pM, quantified using this technique and compared with known senolytics such as 17-DMAG and Ganetespib, which in the cellular model IMR90 seem to have cytotoxic effects already at low concentrations and also in early passages. The compounds were tested in a concentration range between 0.1 and 10 µM for 48 h, appreciating a significant effect starting from the concentration of 0.5 µM. At higher concentrations (> 10 µM), the variability increases and the differences between the two cell populations.

For K5, the *in vitro* affinity to the target protein HSP90α was evaluated using a geldanamycin-competitive binding fluorimetric assay (HSP90α N-Terminal Domain Assay Kit, #50293, BPS Bioscience, Inc., CA, USA). K5 was incubated in a concentration range between 1 pM and 10 µM for 2 h, with a FITC-labeled geldanamycin and the recombinant HSP90α enzyme to produce a change in fluorescent polarization, measured by the microplate multimode reader, Victor NIVO (PerkinElmer Tech. GmbH, Germany). The resulting data allowed the calculation of the compound IC50s, both in the nanomolar order (K5=731 nm); 17-DMAG was used as the reference molecule (IC50=11nM).

IC50 or 50 inhibiting concentration is the concentration of a compound required to inhibit by 50% the target activity under examination with respect to the values measured in the absence of inhibitor. IC50 is a parameter used to evaluate the effectiveness of a substance in inhibiting the target and it is one of the methods commonly used in the pharmacological research to measure the power of an antagonist.

A test was carried out to compare the binding affinities for HSP90α of the compounds of formula (Ia) to (Il) according to the invention.

Table 2 below summarises the results.

**Table 2 - Binding affinities for HSP90α of the compounds of formula (Ia) to (Il)**

| Compound | Binding affinity (Kcal/mol) |
|---|---|
| Ia (K5) | -4.964 |
| Ib | -7.029 |
| Ic | -6.607 |
| Id | -5.675 |
| Ie | -5.502 |
| If | -5.083 |
| Ig | -5.041 |
| Ih | -4.974 |
| Ii | -4.872 |
| Il | -4.857 |

Binding affinities for HSP90 for all compounds of formula (I) were calculated by the program Glide (Schrödinger, Inc. )

### EXAMPLE 2

### Synthesis of the compound 1-benzyl-3-(2-methylphenyl)-2,3,6,7-tetrahydro-1H-purine-2,6-dione(K5)

The synthesis took place according to the following scheme:

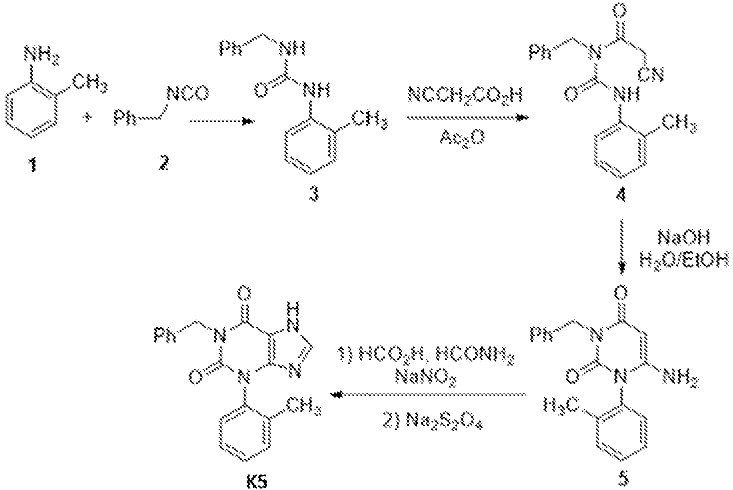

Ac₂O is acetic anhydride; EtOH is ethanol.

Compounds 1 and 2 were purchased from Sigma-Aldrich (US) (www.sigmaaldrich.com) (CAS numbers 95-53-4 and 3173-56-6, respectively).

The condensation reaction of o-toluidine (1) (CAS number 95-53-4) with benzyl isocyanate (2) (CAS number 3173-56-6) gives urea (3) whose acylation with cyanoacetic acid, provides acylurea (4). Hydrolysis of the cyano group, followed by in situ cyclization, yields aminouracyl (5) (see Papesch V, Schroder E.F., J. Org. Chem. 1951, 16, 1879; Ohtsuka Y., Bull. Soc. Chim. Jap. 1973, 46, 506). Nitrosation of (5) and reduction (sodium dithionite/formamide) of the corresponding nitroso derivative to amine and reaction of the resulting diamine intermediate with formic acid, gives the compound of formula (Ia) (K5) (see Brodereck H., Edenhofer A., Chem. Berichte 1955, 1306; Montana J.G. et al., Bioorg. Med. Chem. 1998, 8, 2925).

## Claims

1. A compound of formula (I) or pharmaceutically acceptable salts or hydrates thereof: 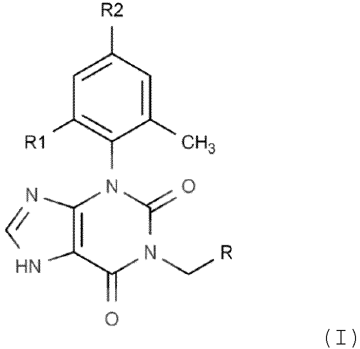
wherein R is chosen from
- phenyl, a 6-membered heteroaryl group, cyclohexyl, a 5-membered heteroaryl group;
- a bicyclo [3.1.0] hexanyl group;
- a C₂-C₅ alkynyl group; and
- a cubanyl group;
wherein R1 and R2, independently, are chosen from
- H;
- C₁₋C₆ alkyl (optionally substituted with one or more halogens) ;
- C₁-C₆ alkyl-S(O)ₙ-;
- -CO₂H (or C₁-C₆ alkyl esters thereof or C₁-C₆ alkyl amides thereof);
- halogen;
- C₁-C₆ alkoxy;
- CN;
- NO₂; and
- NR₇R₈;
wherein R₇ and R₈, independently, each represent H, C₁-C₆ alkyl, C₁-C₆ alkylcarbonyl, C₁-C₆ alkoxycarbonyl, arylsulphonyl, heteroarylsulphonyl, heterocyclosulphonyl, arylcarbonyl, heteroarylcarbonyl, heterocyclocarbonyl, or C₁-C₆ alkylsulphonyl, or R₇, R₈ and the nitrogen to which they are attached form a 5 or 6 membered heterocyclic ring (such as morpholine or piperidine); and
- n represents 0-2;
for use in the treatment and/or prevention of a senescence-associated disease or disorder, or for therapeutic use in delaying aging in a subject.

2. The compound of formula (I) or pharmaceutically acceptable salts or hydrates thereof according to claim 1, for use according to claim 1, wherein, in R, said 6-membered heteroaryl group or 5-membered heteroaryl group has 1 or 2 heteroatoms, preferably independently selected from N and S.

3. The compound of formula (I) or pharmaceutically acceptable salts or hydrates thereof according to claim 1 or 2, for use according to claim 1, wherein R1 and R2 are independently chosen from -H, -CH₃ and -CH₂CH₃, more preferably R1 and R2 are both -H.

4. The compound of formula (I) or pharmaceutically acceptable salts or hydrates thereof according to any one of claims 1 to 3, for use according to claim 1, wherein R is chosen from the following groups of formula (a), (b), (c), (d), (e), (f), (g), (h), (i), (1) : 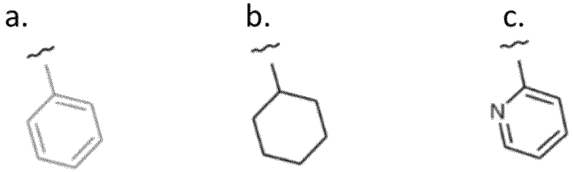 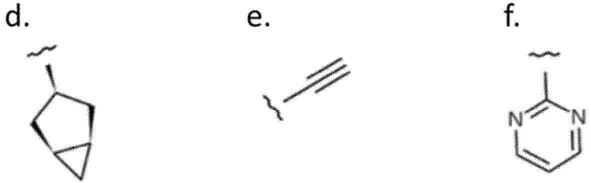 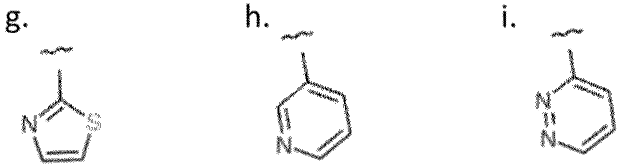 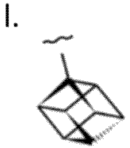 , preferably R is the group of formula (a):

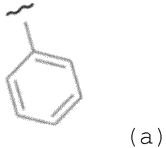

5. The compound of formula (I) or pharmaceutically acceptable salts or hydrates thereof according to any one of claims 1 to 4, for use according to claim 1, wherein the compound of formula (I) is the compound of formula (Ia):

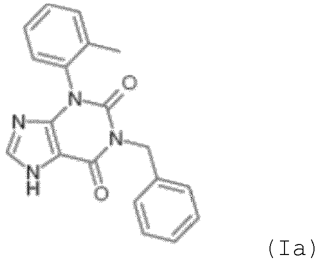

6. A pharmaceutical composition comprising, as active ingredient, at least one compound of formula (I) according to any one of claims 1 to 5, or pharmaceutically acceptable salts or hydrates thereof, and at least one pharmaceutically acceptable excipient for use in the treatment and/or prevention of a senescence-associated disease or disorder, or for therapeutic use in delaying aging in a subject.

7. The compound of formula (I) or pharmaceutically acceptable salts or hydrates thereof according to any one of claims 1 to 5, for use according to claim 1, or the pharmaceutical composition for use according to claim 6, as HSP90 inhibitor, more preferably HSP90α inhibitor.

8. The compound of formula (I) or pharmaceutically acceptable salts or hydrates thereof according to any one of claims 1 to 5, for use according to claim 1, or the pharmaceutical composition for use according to claim 6, having the effect of selectively killing senescent cells.

9. The compound of formula (I) or pharmaceutically acceptable salts or hydrates thereof according to any one of claims 1 to 5, for use according to claim 1, or the pharmaceutical composition for use according to claim 6, wherein said senescence-associated disease or disorder is chosen from a tumour, a metabolic disease, an inflammatory disease or disorder, including an autoimmune disease or disorder, a neurological or neurodegenerative disease or disorder, a pulmonary or respiratory disease or disorder, a proliferative disorder, a renal disorder or disease, a liver disorder or disease, an eye disease or disorder, a cardiovascular disease or disorder, a fibrotic disease or disorder, a viral infection, a dermatological disorder or disease, a geriatric disease or disorder, and side effects associated with chemotherapy or radiotherapy.

10. Cosmetic use of a compound of formula (I) or cosmetically acceptable salts or hydrates thereof: 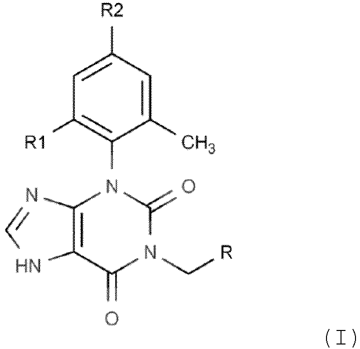
wherein R is chosen from
- phenyl, a 6-membered heteroaryl group, cyclohexyl, a 5-membered heteroaryl group;
- a bicyclo [3.1.0] hexanyl group;
- a C₂-C₅ alkynyl group; and
- a cubanyl group;
wherein R1 and R2, independently, are chosen from
- H;
- C₁-C₆ alkyl (optionally substituted with one or more halogens) ;
- C₁-C₆ alkyl-S(O)ₙ-;
- -CO₂H (or C₁-C₆ alkyl esters thereof or C₁-C₆ alkyl amides thereof);
- halogen;
- C₁-C₆ alkoxy;
- CN;
- NO₂; and
- NR₇R₈;
wherein R₇ and R₈, independently, each represent H, C₁-C₆ alkyl, C₁-C₆ alkylcarbonyl, C₁-C₆ alkoxycarbonyl, arylsulphonyl, heteroarylsulphonyl, heterocyclosulphonyl, arylcarbonyl, heteroarylcarbonyl, heterocyclocarbonyl, or C₁-C₆ alkylsulphonyl, or R₇, R₈ and the nitrogen to which they are attached form a 5 or 6 membered heterocyclic ring (such as morpholine or piperidine); and
- n represents 0-2;
to prevent, reduce and/or treat the signs of ageing of the skin.

11. Cosmetic use according to claim 10, wherein the compound of formula (I) is the compound of formula (Ia):

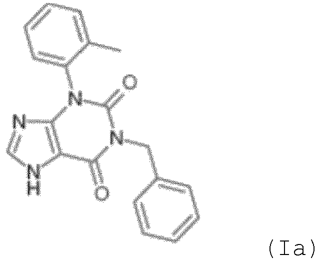

12. Cosmetic use of a cosmetic composition comprising at least one compound of formula (I) or cosmetically acceptable salts or hydrates thereof of claims 10 or 11, and at least one cosmetically acceptable excipient to prevent, reduce and/or treat the signs of ageing of the skin. .

13. Cosmetic use of the cosmetic composition of claim 12 to prevent, reduce and/or treat the signs of ageing of the skin.

## Patentansprüche

1. Eine Verbindung der Formel (I) oder pharmazeutisch akzeptable Salze oder Hydrate davon: 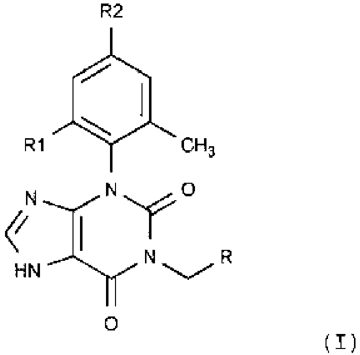
wobei R ausgewählt ist aus
- Phenyl, einer 6-gliedrigen Heteroarylgruppe, Cyclohexyl, einer 5-gliedrigen Heteroarylgruppe;
- einer Bicyclo[3.1.0]hexanylgruppe;
- einer C₂-C₅ Alkinylgruppe; und
- einer Cubanylgruppe;
wobei R1 und R2 unabhängig voneinander ausgewählt sind aus
- H;
- C₁-C₆ Alkyl (gegebenenfalls substituiert mit einem oder mehreren Halogenen);
- C₁-C₆ Alkyl -S(O)ₙ-;
- -CO₂H (oder C₁-C₆ Alkylester davon oder C₁-C₆ Alkylamide davon);
- Halogen;
- C₁-C₆ Alkoxy;
- CN;
- NO₂; und
- NR₇R₈;
wobei R₇ und R₈, unabhängig voneinander, jeweils H, C₁-C₆ Alkyl, C₁-C₆ Alkylcarbonyl, C₁-C₆ Alkoxycarbonyl, Arylsulphonyl, Heteroarylsulphonyl, Heterocyclosulphonyl, Arylcarbonyl, Heteroarylcarbonyl, Heterocyclocarbonyl oder C₁-C₆ Alkylsulphonyl darstellen, oder R₇, R₈ und der Stickstoff, an den sie gebunden sind, einen 5 oder 6 gliedrigen heterocyclischen Ring bilden (wie Morpholin oder Piperidin); und
- n 0-2 darstellt;
zur Verwendung bei der Behandlung und/oder Vorbeugung einer seneszenzassoziierten Krankheit oder Störung oder zur therapeutischen Verwendung bei der Verzögerung der Alterung bei einem Subjekt.

2. Die Verbindung der Formel (I) oder pharmazeutisch akzeptable Salze oder Hydrate davon nach Anspruch 1, zur Verwendung nach Anspruch 1, wobei in R die genannte 6-gliedrige Heteroarylgruppe oder 5-gliedrige Heteroarylgruppe 1 oder 2 Heteroatome aufweist, vorzugsweise unabhängig ausgewählt aus N und S.

3. Die Verbindung der Formel (I) oder pharmazeutisch akzeptable Salze oder Hydrate davon nach Anspruch 1 oder 2, zur Verwendung nach Anspruch 1, wobei R1 und R2 unabhängig ausgewählt sind aus -H, -CH₃ und -CH₂CH₃, bevorzugter sind R1 und R2 beide -H.

4. Die Verbindung der Formel (I) oder pharmazeutisch akzeptable Salze oder Hydrate davon nach einem der Ansprüche 1 bis 3, zur Verwendung nach Anspruch 1, wobei R ausgewählt ist aus den folgenden Gruppen der Formel (a), (b), (c), (d), (e), (f), (g), (h), (i), (l): 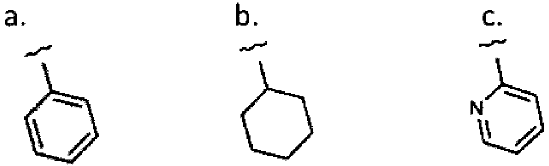 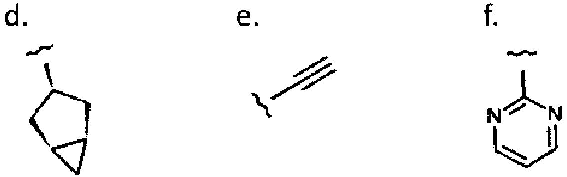 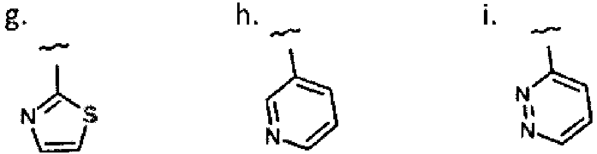 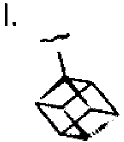 , vorzugsweise ist R die Gruppe der Formel (a):

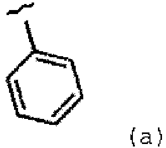

5. Die Verbindung der Formel (I) oder pharmazeutisch akzeptable Salze oder Hydrate davon nach einem der Ansprüche 1 bis 4, zur Verwendung nach Anspruch 1, wobei die Verbindung der Formel (I) die Verbindung der Formel (Ia) ist:

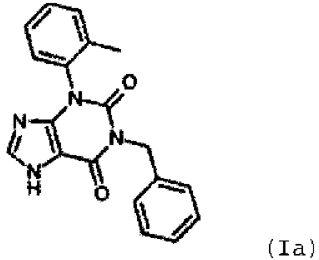

6. Eine pharmazeutische Zusammensetzung, die als Wirkstoff mindestens eine Verbindung der Formel (I) nach einem der Ansprüche 1 bis 5 oder pharmazeutisch akzeptable Salze oder Hydrate davon und mindestens einen pharmazeutisch akzeptablen Hilfsstoff umfasst, zur Verwendung bei der Behandlung und/oder Vorbeugung einer seneszenzassoziierten Krankheit oder Störung oder zur therapeutischen Verwendung bei der Verzögerung der Alterung bei einem Subjekt.

7. Die Verbindung der Formel (I) oder pharmazeutisch akzeptable Salze oder Hydrate davon nach einem der Ansprüche 1 bis 5, zur Verwendung nach Anspruch 1, oder die pharmazeutische Zusammensetzung zur Verwendung nach Anspruch 6, als HSP90-Inhibitor, bevorzugter HSP90α-Inhibitor.

8. Die Verbindung der Formel (I) oder pharmazeutisch akzeptable Salze oder Hydrate davon nach einem der Ansprüche 1 bis 5, zur Verwendung nach Anspruch 1, oder die pharmazeutische Zusammensetzung zur Verwendung nach Anspruch 6, die die Wirkung hat, seneszente Zellen selektiv abzutöten.

9. Die Verbindung der Formel (I) oder pharmazeutisch akzeptable Salze oder Hydrate davon nach einem der Ansprüche 1 bis 5, zur Verwendung nach Anspruch 1, oder die pharmazeutische Zusammensetzung zur Verwendung nach Anspruch 6, wobei die genannte seneszenzassoziierte Krankheit oder Störung ausgewählt ist aus einem Tumor, einer Stoffwechselerkrankung, einer entzündlichen Krankheit oder Störung, einschließlich einer Autoimmunkrankheit oder -störung, einer neurologischen oder neurodegenerativen Krankheit oder Störung, einer Lungen- oder Atemwegserkrankung oder -störung, einer proliferativen Störung, einer Nierenstörung oder -erkrankung, einer Leberstörung oder -erkrankung, einer Augenkrankheit oder -störung, einer kardiovaskulären Krankheit oder Störung, einer fibrotischen Krankheit oder Störung, einer Virusinfektion, einer dermatologischen Störung oder Erkrankung, einer geriatrischen Krankheit oder Störung und Nebenwirkungen, die mit Chemotherapie oder Strahlentherapie verbunden sind.

10. Kosmetische Verwendung einer Verbindung der Formel (I) oder kosmetisch akzeptabler Salze oder Hydrate davon: 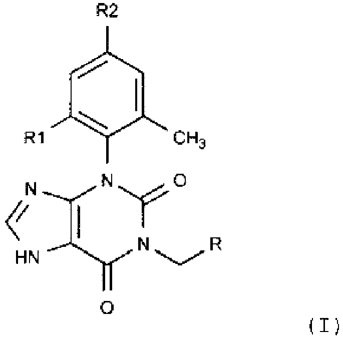
wobei R ausgewählt ist aus
- Phenyl, einer 6-gliedrigen Heteroarylgruppe, Cyclohexyl, einer 5-gliedrige Heteroarylgruppe:
- eine Bicyclo [3.1.0] hexanylgruppe:
- einer C₂-C₅ Alkinylgruppe; und
- einer Cubanylgruppe;
wobei R1 und R2 unabhängig voneinander ausgewählt sind aus
- H;
- C₁-C₆ Alkyl (gegebenenfalls substituiert mit einem oder mehreren Halogenen);
- C₁-C₆ Alkyl -S(O)ₙ-;
- -CO₂H (oder C₁-C₆ Alkylester davon oder C₁-C₆ Alkylamide davon);
- Halogen;
- C₁-C₆ Alkoxy;
- CN;
- NO₂; und
- NR₇R₈;
wobei R₇ und R₈, unabhängig voneinander, jeweils H, C₁-C₆ Alkyl, C₁-C₆ Alkylcarbonyl, C₁-C₆ Alkoxycarbonyl, Arylsulphonyl, Heteroarylsulphonyl, Heterocyclosulphonyl, Arylcarbonyl, Heteroarylcarbonyl, Heterocyclocarbonyl oder C₁-C₆ Alkylsulphonyl darstellen, oder R₇, R₈ und der Stickstoff, an den sie gebunden sind, einen 5- oder 6-gliedrigen heterocyclischen Ring bilden (wie Morpholin oder Piperidin); und
- n 0-2 darstellt;
um die Zeichen der Hautalterung zu verhindern, zu reduzieren und/oder zu behandeln.

11. Kosmetische Verwendung nach Anspruch 10, wobei die Verbindung der Formel (I) die Verbindung der Formel (la) ist:

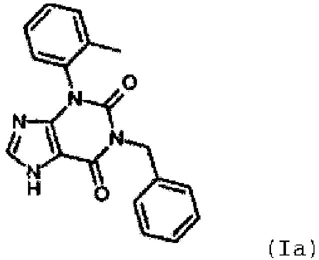

12. Kosmetische Verwendung einer kosmetischen Zusammensetzung, die mindestens eine Verbindung der Formel (I) oder kosmetisch akzeptable Salze oder Hydrate davon nach Anspruch 10 oder 11 und mindestens einen kosmetisch akzeptablen Hilfsstoff umfasst, um die Zeichen der Hautalterung zu verhindern, zu reduzieren und/oder zu behandeln.

13. Kosmetische Verwendung der kosmetischen Zusammensetzung nach Anspruch 12, um die Zeichen der Hautalterung zu verhindern, zu reduzieren und/oder zu behandeln.

## Revendications

1. Composé de formule (I) ou ses sels ou hydrates pharmaceutiquement acceptables: 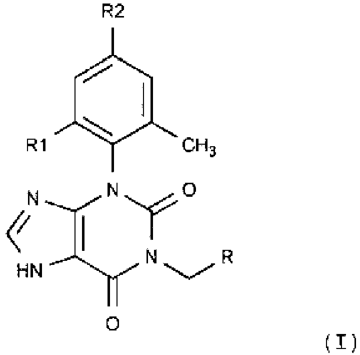
dans lequel R est choisi parmi
- phényle, un groupe hétéroaryle à 6 chaînons, cyclohexyle, un groupe hétéroaryle à 5 chaînons;
- un groupe bicyclo [3.1.0] hexanyle;
- un groupe alcynyle en C₂-C₅; et
- un groupe cubanyle;
dans lequel R1 et R2, indépendamment, sont choisis parmi
- H;
- alkyle en C₁-C₆ (éventuellement substitué par un ou plusieurs halogènes);
- alkyle en C₁-C₆ -S(O)ₙ-;
- -CO₂-H (ou ses esters d'alkyle en C₁-C₆ ou ses amides d'alkyle en C₁-C₆);
- halogène;
- alcoxy en C₁-C₆;
- CN;
- NO₂; et
- NR₇R₈;
dans lequel R₇ et R₈, indépendamment, représentent chacun H, alkyle en C₁-C₆, alkylcarbonyle en C₁-C₆, alcoxycarbonyle en C₁-C₆, arylsulfonyle, hétéroarylsulfonyle, hétérocyclosulfonyle, arylcarbonyle, hétéroarylcarbonyle, hétérocyclocarbonyle, ou alkylsulfonyle en C₁-C₆, ou R₇, R₈ et l'azote auquel ils sont attachés forment un cycle hétérocyclique à 5 ou 6 chaînons (tel que morpholine ou pipéridine); et
- n représente 0-2;
pour une utilisation dans le traitement et/ou la prévention d'une maladie ou d'un trouble associé à la sénescence, ou pour une utilisation thérapeutique dans le retardement du vieillissement chez un sujet.

2. Composé de formule (I) ou ses sels ou hydrates pharmaceutiquement acceptables selon la revendication 1, pour une utilisation selon la revendication 1, dans lequel, dans R, ledit groupe hétéroaryle à 6 chaînons ou groupe hétéroaryle à 5 chaînons possède 1 ou 2 hétéroatomes, de préférence indépendamment choisis parmi N et S.

3. Composé de formule (I) ou ses sels ou hydrates pharmaceutiquement acceptables selon la revendication 1 ou 2, pour une utilisation selon la revendication 1, dans lequel R1 et R2 sont indépendamment choisis parmi -H, -CH₃ et -CH₂CH₃, plus préférablement R1 et R2 sont tous les deux -H.

4. Composé de formule (I) ou ses sels ou hydrates pharmaceutiquement acceptables selon l'une quelconque des revendications 1 à 3, pour une utilisation selon la revendication 1, dans lequel R est choisi parmi les groupes suivants de formule (a), (b), (c), (d), (e), (f), (g), (h), (i), (1): 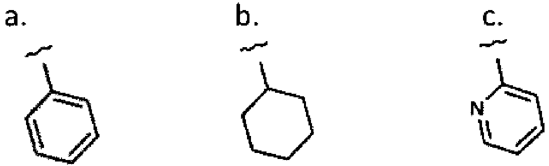 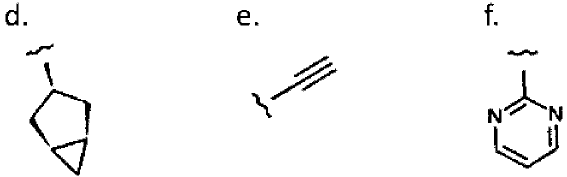 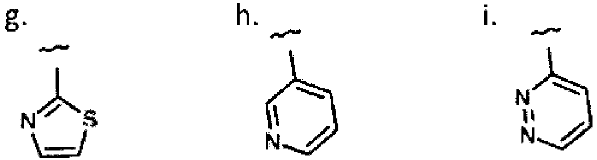 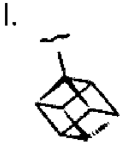 , de préférence R est le groupe de formule (a):

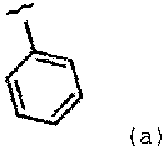

5. Composé de formule (I) ou ses sels ou hydrates pharmaceutiquement acceptables selon l'une quelconque des revendications 1 à 4, pour une utilisation selon la revendication 1, dans lequel le composé de formule (I) est le composé de formule (la):

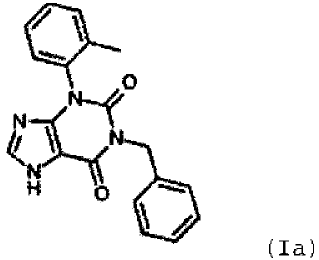

6. Composition pharmaceutique comprenant, comme ingrédient actif, au moins un composé de formule (I) selon l'une quelconque des revendications 1 à 5, ou ses sels ou hydrates pharmaceutiquement acceptables, et au moins un excipient pharmaceutiquement acceptable pour une utilisation dans le traitement et/ou la prévention d'une maladie ou d'un trouble associé à la sénescence, ou pour une utilisation thérapeutique dans le retardement du vieillissement chez un sujet.

7. Composé de formule (I) ou ses sels ou hydrates pharmaceutiquement acceptables selon l'une quelconque des revendications 1 à 5, pour une utilisation selon la revendication 1, ou composition pharmaceutique pour une utilisation selon la revendication 6, comme inhibiteur de HSP90, plus préférablement inhibiteur de HSP90α.

8. Composé de formule (I) ou ses sels ou hydrates pharmaceutiquement acceptables selon l'une quelconque des revendications 1 à 5, pour une utilisation selon la revendication 1, ou composition pharmaceutique pour une utilisation selon la revendication 6, ayant l'effet de tuer sélectivement les cellules sénescentes.

9. Composé de formule (I) ou ses sels ou hydrates pharmaceutiquement acceptables selon l'une quelconque des revendications 1 à 5, pour une utilisation selon la revendication 1, ou composition pharmaceutique pour une utilisation selon la revendication 6, dans lequel ladite maladie ou ledit trouble associé à la sénescence est choisi parmi une tumeur, une maladie métabolique, une maladie ou un trouble inflammatoire, y compris une maladie ou un trouble auto-immun, une maladie ou un trouble neurologique ou neurodégénératif, une maladie ou un trouble pulmonaire ou respiratoire, un trouble prolifératif, un trouble ou une maladie rénale, un trouble ou une maladie hépatique, une maladie ou un trouble oculaire, une maladie ou un trouble cardiovasculaire, une maladie ou un trouble fibrotique, une infection virale, un trouble ou une maladie dermatologique, une maladie ou un trouble gériatrique, et les effets secondaires associés à la chimiothérapie ou à la radiothérapie.

10. Utilisation cosmétique d'un composé de formule (I) ou de ses sels ou hydrates cosmétiquement acceptables: 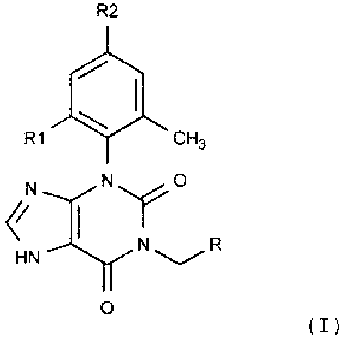
dans lequel R est choisi parmi
- phényle, un groupe hétéroaryle à 6 chaînons, cyclohexyle, un groupe hétéroaryle à 5 chaînons:
- un groupe bicyclo [3.1.0] hexanyle:
- un groupe alcynyle en C₂-C₅; et
- un groupe cubanyle;
dans lequel R1 et R2, indépendamment, sont choisis parmi
- H;
- alkyle en C₁-C₆ (éventuellement substitué par un ou plusieurs halogènes);
- alkyle en C₁-C₆ -S (O)ₙ-;
- -CO₂H (ou ses esters d'alkyle en C₁-C₆ ou ses amides d'alkyle en C₁-C₆);
- halogène;
- alcoxy en C₁-C₆;
- CN;
- NO₂; et
- NR₇R₈;
dans lequel R₇ et R₈, indépendamment, représentent chacun H, alkyle en C₁-C₆, alkylcarbonyle en C₁-C₆, alcoxycarbonyle en C₁-C₆, arylsulfonyle, hétéroarylsulfonyle, hétérocyclosulfonyle, arylcarbonyle, hétéroarylcarbonyle, hétérocyclocarbonyle, ou alkylsulfonyle en C₁-C₆, ou R₇, R₈ et l'azote auquel ils sont attachés forment un cycle hétérocyclique à 5 ou 6 chaînons (tel que morpholine ou pipéridine); et
- n représente 0-2;
pour prévenir, réduire et/ou traiter les signes du vieillissement de la peau.

11. Utilisation cosmétique selon la revendication 10, dans laquelle le composé de formule (I) est le composé de formule (la):

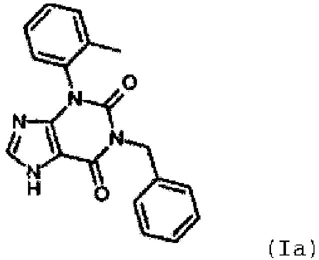

12. Utilisation cosmétique d'une composition cosmétique comprenant au moins un composé de formule (I) ou de ses sels ou hydrates cosmétiquement acceptables selon les revendications 10 ou 11, et au moins un excipient cosmétiquement acceptable pour prévenir, réduire et/ou traiter les signes du vieillissement de la peau.

13. Utilisation cosmétique de la composition cosmétique selon la revendication 12 pour prévenir, réduire et/ou traiter les signes du vieillissement de la peau.
